(19) Europäisches Patentamt
European Patent Office
Office européen des brevets

(11) **EP 1 586 888 A2**

(12) **EUROPEAN PATENT APPLICATION**

(43) Date of publication:
**19.10.2005 Bulletin 2005/42**

(51) Int Cl.[7]: **G01N 21/27**, G01N 21/31,
G01N 33/48, B01L 3/00

(21) Application number: **05252270.3**

(22) Date of filing: **12.04.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(30) Priority: **14.04.2004 US 823778**

(71) Applicant: **Spectromedical Inc.**
**Cambridge, Ontario N1T 1M7 (CA)**

(72) Inventor: **Samsoondar, James**
**Cambridge Ontario N1T 1M7 (CA)**

(74) Representative: **Crease, Devanand John et al**
**David Keltie Associates**
**Patent and Trade Mark Attorneys**
**Fleet Place House**
**2 Fleet Place**
**London EC4M 7ET (GB)**

(54) **Spectroscopic method and apparatus for analyte measurement**

(57)  An apparatus and method for spectroscopic measurement of an analyte in a sample is provided. The apparatus comprises a source of electromagnetic radiation (EMR) producing a light path, an aperture located within the light path and between the EMR source and a sample slot, and a photodector. The apparatus also has a primary calibration algorithm that is in operative association with the spectroscopic apparatus. Examples of analytes that may be measured using this apparatus include, but are not limited to Total-Hemoglobin, Met-Hemoglobin, Hemoglobin-based blood substitutes and any Met-Hemoglobin equivalent. The measurement of Met-Hemoglobin may be used to provide an accurate measurement of Total-Hemoglobin in whole blood, or Hemoglobin when used as an indicator of hemolysis. The measurement of Met-Hemoglobin may also be also used as a means of monitoring the degradation or reversal of degradation of Hemoglobin-based blood substitutes, or as a means of monitoring the oxidation or reversal of oxidation of Hemoglobin to Met-Hemoglobin.

Figure 4a

EP 1 586 888 A2

**Description**

This application is a continuation-in-part of application of US serial number 10/805,290 filed on March 22, 2004, which is a continuation of US serial number 09/875,143, filed on June 7,2001, (issued as US patent number 6,711,516), which is a continuation-in-part of application US serial number 09/773,495 filed on February 2, 2001, (abandoned), which is a continuation-in-part of US serial number 09/697,679 filed on October 27, 2000, (abandoned), which is a continuation-in-part of US serial number 09/447,215 filed on November 23, 1999, (issued as US patent number 6,470,279).

This application is also a continuation-in-part of US 10/319,492 filed on July 3, 2003, which is a continuation-in-part application of US serial number 10/023,869 filed December 21, 2001, which is a continuation-in-part ofUS serial number 09/147,373 filed June 12, 1997, (issued as US patent number 6,372,503 B1), which claims priority from GB serial number 9612264.3 filed June 12, 1996.

This application is also a continuation-in-part of 10/136,329 filed on May 2, 2002, which is a continuation-in-part of US serial number 10/023,869 filed on December 21, 2001.

This application is also a continuation-in-part of 10/042,258 filed on January 11, 2002, which is a continuation-in-part of US serial number 09/958,933 filed on May 11, 2000, (issue as US patent number 6,582,964), which claims priority to US serial number 60/133,876 filed on May 12, 1999.

**FIELD OF INVENTION**

This invention relates to the field of spectroscopic measurements of analytes in biological samples. More specifically, the invention relates to a method and apparatus used for Hemoglobin (Hb) measurement, and substances related to Hb.

Clinical laboratory tests are routinely performed on the serum or plasma of whole blood. In a routine assay, red blood cells (RBC) are separated from plasma by centrifugation, or RBC's and various plasma proteins are separated from serum by clotting prior to centrifugation. Hb, light-scattering substances like lipid particles, and bile pigments bilirubin (BR) and biliverdin (BV) are typical blood components, which will interfere with and affect spectroscopic and other blood analytical measurements of blood analytes. Such components are referred to as interferents, and they can be measured by spectroscopic methods. The presence of such interferents affects the ability to perform tests on the serum or plasma and as such can be said to compromise sample integrity.

Current methods of measuring Total-Hemoglobin (Tot-Hb) in a sample, preferably use reagents, whereby the different Hb species like Oxy-hemoglobin (Oxy-Hb), Deoxy-Hemoglobin (Deoxy-Hb), Carboxy-Hemoglobin (Carboxy-Hb), and Met-Hemoglobin (Met-Hb) are converted to a single specie, which is then measured at one wavelength using spectroscopic methods; sometimes a second wavelength is also used. The reagents are usually noxious (e.g. potassium cyanide and azide), and there is a need for a reagentless method for measuring Hb in body fluids. Harboe (Harboe, M., 1959, A method of determination of hemoglobin in plasma by near ultraviolet spectrophotometry. Scand. J. Clin. Lab. Invest, pp. 66-70) and Tietz (Tietz Textbook of Clinical Chemistry, 3rd Ed, 1999, pp 1674-1676; which is incorporated herein by reference), provide examples of reagentless spectroscopic methods for measuring Hb. Although Hb provides very large absorbance signals, the absorbance spectra of the Hb species exhibit significant differences. Reagentless spectroscopic methods are limited to samples that contain mostly Oxy-Hb and Deoxy-Hb. The Deoxy-Hb is usually converted into Oxy-Hb when the sample is exposed briefly to atmospheric oxygen. The largest source of errors in both methods (Harboe & Tietz) is the presence of Met-Hb. In US 6,689,612 (Samsoondar), there is described the use of Total-Hb, Oxy-Hemoglobin (Oxy-Hb), and "Total-Hb minus Met-Hemoglobin (Met-Hb)," as indicators of hemolysis. Because the absorbance spectrum for Met-Hb is so different from the other Hb species, a calibration algorithm developed for Hb may be better at predicting "Total-Hb minus Met-Hb."

Met-Hb is an oxidation product of Hb and the Met-Hb form of the Hemoglobin-based (Hb-based) blood substitutes is also an oxidation product of Hemoglobin-based blood substitutes. Met-Hb from natural or Hb-based blood substitutes cannot carry oxygen, and therefore is not useful Hb.

**SUMMARY OF THE INVENTION**

This invention relates to the field of spectroscopic measurements of analytes in biological samples. More specifically, the invention relates to a method and apparatus used for Hemoglobin (Hb) measurement, and substances related to Hb.

It is an object of the invention to provide an improved method and apparatus for analyte measurement.

According to the present invention there is provided a method (A) for measuring Corrected Total-Hemoglobin (Corr-Total-Hb) in a sample, comprising:

i) developing a first primary calibration algorithm for one or more than one of Total-Hb, Oxy-Hemoglobin (Oxy-Hb), and "Total Hemoglobin minus Met-hemoglobin" (Total-Hb minus Met-Hb), for predicting a concentration for one or more than one of the Total-Hb, Oxy-Hb, and "Total-Hb minus Met-Hb," in the sample, and producing a first value;

ii) deriving a second primary calibration algorithm for Met-Hb, for predicting a concentration of the Met-Hb in the sample and producing a second value; and

iii) measuring the sample using a spectroscopic apparatus to obtain the first and second values;

iv) adding either:

- the second value to the first value to produce the Corrected Total-Hemoglobin; or

- terms of the first primary calibration algorithm for one of the one or more than one of Total-Hb, Oxy-Hb, and "Total-Hb minus Met-Hb," to terms of the second primary calibration algorithm to produce a single set of terms for a single calibration algorithm, which measures the Corrected Total-Hemoglobin.

Each of the Total-Hb, Oxy-Hb, "Total-Hb minus Met-Hb," or Corr-Total-Hb, may be used as an indicator of hemolysis.

The present invention also pertains to a method (B) for measuring Corrected Total-Hemoglobin (Corr-Total-Hb) in a sample, comprising:

i) providing a spectroscopic apparatus comprising one or more than one first primary calibration algorithm and a second primary calibration algorithm, the first primary calibration algorithm developed for one or more than one of Total-Hb, Oxy-Hemoglobin (Oxy-Hb), and "Total Hemoglobin minus Met-hemoglobin" (Total-Hb minus Met-Hb), for predicting a concentration for one or more than one of the Total-Hb, Oxy-Hb, and "Total-Hb minus Met-Hb," in the sample, thereby producing a first value, and the second primary calibration algorithm developed for Met-Hb, for predicting a concentration of the Met-Hb in the sample and producing a second value; and

ii) measuring the sample using a spectroscopic apparatus to obtain the first and second values;

iii) adding either:

- the second value to the first value to produce the Corrected Total-Hemoglobin; or

- terms of the first primary calibration algorithm for one of the one or more than one of Total-Hb, Oxy-Hb, and "Total-Hb minus Met-Hb," to terms of the second primary calibration algorithm to produce a single set of terms for a single calibration algorithm, which measures the Corrected Total-Hemoglobin.

In the method (B) described above, each of the Total-Hb, Oxy-Hb, "Total-Hb minus Met-Hb," or Corr-Total-Hb, may be used as an indicator of hemolysis.
The present invention pertains to either method (A) or (B) described above wherein the step of developing, deriving, or providing, each comprise the steps of:

a) collecting an absorbance measurement at one or more than one wavelength of a standard set of wavelengths, for each calibration sample in a primary calibration set, the each calibration sample having a known reference value for one or more of the Total-Hb, Oxy-Hb, Met-Hb, and "Total-Hb minus Met-Hb;" and

b) creating the first primary calibration algorithm for one or more than one of the Total-Hb, Oxy-Hb, "Total-Hb minus Met-Hb"; and the Met-Hb, using an order derivative of absorbance, the known reference value for each calibration sample, and a statistical technique. Preferably, the statistical technique is selected from the group consisting of simple linear regression, multiple linear regression, and multivariate analysis. Furthermore, the multivariate analysis is selected from the group consisting of partial least squares, principal component analysis, neural network, and genetic algorithm. Additionally, the one or more than one wavelengths comprises wavelengths selected from a range of wavelengths from about 300 nm to about 2500 nm or any amount therebetween, or from about 450 nm to about 1100 nm, or any amount therebetween.

The preset invention embraces either method (A) or (B) described above wherein in the step of collecting (step a)), the known reference value for either the Total-Hb or Oxy-Hb, is obtained from measured amounts of the Total-Hb or Oxy-Hb, in the presence of one or more than one of Oxy-Hb, Deoxy-Hb, Carboxy-Hb and Met-Hb in the calibration samples. If the Oxy-Hb accounts for about 95% ofTotal-Hb in the sample, or the Total-Hb in the sample may comprise about 95% Oxy-Hb, the value of the Total-Hb and the value of Oxy-Hb could be regarded as being the same, by approximation.

The present invention includes either method (A) or (B) described above wherein the sample is selected from the group consisting of whole blood, serum, plasma, urine, synovial fluid, lymphatic fluid, sputum, feces and cerebrospinal fluid.

Preferably, the spectroscopic apparatus used in either method (A) or (B) described above comprises:

a) one or more than one source of electromagnetic radiation (EMR) that produce a light path;

b) one or more than one photodetector in alignment with the light path;

c) a slot for receiving a sample vessel to be placed within the light path; and

d) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

Furthermore, the sample vessel maybe selected from the group consisting of, a cuvette, a sample tab, a pipette tip, tubing, a labeled test tube, an unlabeled test tube, blood bag tubing, a transparent sample container, a translucent sample container, and a flow-through cuvette.

The present invention also provides a method (C) for flagging a predicted value for an indicator of hemolysis in a sample, or a predicted value for Total-Hb in a sample, for the presence of Met-Hb, the method comprising:

i) providing a spectroscopic apparatus comprising a first primary calibration algorithm for one or more than one of Total-Hb, Oxy-Hb, or "Total-Hb minus Met-Hb," for predicting a first value for one or more than one of the Total-Hb, Oxy-Hb, or "Total-Hb minus Met-Hb," in the sample, wherein each of the Total-Hb, the Oxy-Hb, or the "Total-Hb minus Met-Hb," is used as an indicator of hemolysis in the sample, and a second primary calibration algorithm for Met-Hb, for predicting a second value for the Met-Hb in the sample; and

ii) flagging the first value if the second value exceeds a pre-determined value.

Preferably, the sample is selected from the group consisting of whole blood, serum, plasma, urine, synovial fluid, lymphatic fluid, sputum, feces and cerebrospinal fluid.

The present invention includes the method (C) described above wherein the step of providing (step i)), each of the one or more than one primary calibration algorithm and the second primary calibration algorithm is developed comprising the steps of:

a) collecting an absorbance measurement at one or more than one wavelength of a standard set of wavelengths, for each calibration sample in a primary calibration set, the each calibration sample having a known reference value for one or more of the Total-Hb, Oxy-Hb, Met-Hb, "Total-Hb minus Met-Hb;" and

b) creating the first primary calibration algorithm for one or more than one of the Total-Hb, Oxy-Hb, "Total-Hb minus Met-Hb;"and the Met-Hb, using an order derivative of absorbance, the known reference value for each calibration sample, and a statistical technique.

The present invention also pertains to the method (C) described above wherein in the step of collecting (step a)), the reference value for either the Total-Hb or Oxy-Hb, is obtained from measured amounts of the Total-Hb or Oxy-Hb, in the presence of one or more than one of Oxy-Hb, Deoxy-Hb, Carboxy-Hb and Met-Hb in the calibration samples. If the Oxy-Hb accounts for about 95% of Total-Hb in the sample, or the Total-Hb in the sample may comprise about 95% Oxy-Hb, the value of the Total-Hb and the value of Oxy-Hb could be regarded as being the same, by approximation.

The present invention also relates to the method (C) described above wherein in the step of creating (step b)), the statistical technique is selected from the group consisting of simple linear regression, multiple linear regression, and multivariate analysis. Preferably, the multivariate analysis is selected from the group consisting of partial least squares, principal component analysis, neural network, and genetic algorithm. Additionally, the one or more than one wavelengths comprises wavelengths selected from a range of wavelengths from about 300 nm to about 2500 nm or any amount therebetween, or from about 450 nm to about 1100 nm, or any amount therebetween.

The present invention also provides for the method (C) described above, wherein the spectroscopic apparatus comprises:

a) one or more than one source of electromagnetic radiation (EMR) that produce a light path;

b) one or more than one photodetector in alignment with the light path;

c) a slot for a sample vessel to be placed within the light path;

d) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

Preferably, the sample vessel is selected from the group consisting of, a cuvette, a sample tab, a pipette tip, tubing, labelled test tube, unlabeled test tube, blood bag tubing, a transparent sample container, a translucent sample container, and a flow-through cuvette. Furthermore, the source providing the EMR may be characterized as having one or more than one wavelength from about 300nm to about 2500nm, or any wavelength therebetween. Preferably, the wavelength is from about 450nm to about 1100nm, or any amount therebetween.

The present invention also provides a spectroscopic apparatus, comprising:

a) a source of electromagnetic radiation (EMR);

b) a first aperture located between the source of EMR and a sample slot to produce a light path therebetween;

c) the sample slot in the apparatus for receiving a sample vessel to be placed within the light path;

d) a second aperture located in the light path, between the sample slot and one or more than one photodetector, the one or more than one photodetector in operative association with the spectroscopic apparatus; and

e) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

The present invention also embraces a spectroscopic apparatus comprising:

a) a source of electromagnetic radiation (EMR) for producing a light path;

b) an aperture located in the light path between the source of EMR and a sample slot;

c) a sample slot for receiving a sample vessel and placed within the light path, the sample slot comprising a first, and a second, side, the first side facing the source of EMR;

d) a reflective member positioned at or near the second side, the reflective member for reflecting the EMR that passes through the sample slot to produce a reflected light path;

e) one or more than one photodetector located within the reflected light path, the one or more than one photodetector in operative association with the spectroscopic apparatus;

f) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

Additionally, the present invention pertains to a spectroscopic apparatus comprising:

a) a source of electromagnetic radiation (EMR) for producing a light path;

b) an aperture located within the light path between the source of EMR and a sample slot;

c) the sample slot for receiving a sample vessel, and placed within the light path;

d) one or more than one photodetectors located on a same side of the sample slot as the source of EMR, the one or more than one photodetector in operative association with the spectroscopic apparatus;

e) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

Preferably, in each of the above-defined spectroscopic apparatus, the sample vessel is selected from the group consisting of, a cuvette, a sample tab, a pipette tip, tubing, a labeled test tube, an unlabeled test tube, blood bag tubing, a transparent sample container, a translucent sample container, and a flow-through cuvette. Furthermore, the sample tab contains the sample placed between a cover plate and a base plate, wherein at least a portion of the cover plate is reflective, transparent or translucent and at least a portion of the base plate is reflective, transparent or translucent, and wherein the cover plate is hingedly attached to the base plate.

The present invention also includes any of the apparatus as described above wherein the source of EMR is selected from the group consisting of a tungsten lamp, one or more than one Light Emitting Diode (LED), and one or more than one laser. The source providing the EMR may be characterized as having one or more than one wavelength from about 300nm to about 2500nm, or any wavelength therebetween. Preferably, the wavelength is from about 450nm to about 1100nm, or any amount therebetween. Furthermore, the one or more than one photodetector is selected from the group consisting of Photodiode or Charged Coupled Detector (CCD).

Additionally, the one or more than one calibration algorithm may be developed using a statistical technique selected from the group consisting of simple linear regression, multiple linear regression, and multivariate analysis, where the multivariate analysis is selected from the group consisting of partial least squares, principal component analysis, neural network, and genetic algorithm. The one or more than one primary calibration algorithm may be for an analyte selected from the group consisting of a Hb-based blood substitute, Total-Hb, Oxy-Hb, "Total-Hb minus Met-Hb," Met-Hb, bilirubin, biliverdin, methylene blue, and a combination thereof.

The present invention also provides a method (D) of monitoring degradation or reversal of degradation of one or more than one Hb-based blood substitute in a sample, comprising:

i) measuring absorbance of the sample using a spectroscopic apparatus comprising a calibration algorithm for Met-Hb, at one or more than one wavelength of a standard set of wavelengths, to obtain an absorbance;

ii) determming a first concentration of the Met-Hb from the absorbance, by applying the calibration algorithm, to an order derivative of the absorbance;

iii) determining a second concentration of the Met-Hb in the sample at a second time;

where degradation of the one or more than one blood substitute is indicated by an increase in the second concentration compared to the first concentration, and where reversal of degradation of the one or more than one blood substitute is indicated by a decrease in concentration of the second concentration when compared to the first concentration. For example, if the concentration of the Met-Hb is equal to or greater than 3% of the one or more than one Hb-Based blood substitute, than this is an indicator of degradation of the one or more than one Hb-based blood substitute.

The present invention includes the method (D) as described above wherein the sample is selected from the group consisting of, a whole blood sample obtained from a patient infused with one or more than one Hb-based blood substitutes, a serum sample obtained from a patient infused with one or more Hb-based blood substitutes, a plasma sample obtained from a patient infused with one or more Hb-based blood substitutes, and a stock Hb-based blood substitute.

The present invention embraces the method (D) described above wherein in the step of measuring (step (i)), the calibration algorithm is derived using a statistical technique selected from the group consisting of simple linear regression, multiple linear regression, and multivariate analysis. Furthermore, the multivariate analysis may be selected from the group consisting of partial least squares, principal component analysis, neural network, and genetic algorithm. Additionally, the one or more than one wavelengths comprises wavelengths selected from a range of wavelengths from about 300 nm to about 2500 nm or any amount therebetween, or from about 450 nm to about 1100 nm, or any amount therebetween.

The present invention also provides for a method (E) of monitoring degradation or reversal of degradation of one or more Hb-based blood substitutes in a sample comprising:

i) determining a first concentration of Met-Hb, and a first concentration of the one or more than one Hb-based blood substitutes in the sample, by applying a first calibration algorithm for the Met-Hb, and a second calibration algorithm for the one or more than one Hb-based blood substitutes, to an order derivative of absorbance of the sample at one or more wavelength of a standard set of wavelengths;

ii) determining a second concentration of the Met-Hb and a second concentration of the one or more than one Hb-based blood substitutes in the sample at a second time, by applying a first calibration algorithm for the Met-Hb, and a second calibration algorithm for the one or more than one Hb-based blood substitutes, to an order derivative of absorbance of the sample at one or more wavelength of a standard set of wavelengths; and

iii) calculating a first proportion of the one or more than one Hb-based blood substitutes that is in the form of Met-Hb using the first concentration and the first concentration of the one or more than one Hb-based blood substitutes, and calculating a second proportion of the one or more than one Hb-based blood substitutes that is in the form of Met-Hb using the second concentration and the second concentration of the one or more than one Hb-based blood substitutes;

where an increase in the second proportion, when compared to the first proportion is an indication of degradation of the one or more than one blood substitute, and a decrease in the second proportion, when compared to the first proportion is an indication of a reversal of degradation of the one or more than one Hb-based blood substitute, thereby monitoring degradation or reversal of degradation of the one or more Hb-based blood substitutes.

Also provided in the present invention is a method (F) of determining degradation of one or more than one Hb-based blood substitute in a sample, comprising:

i) measuring an absorbance of the sample at one or more than one wavelengths of a standard set of wavelengths using a spectroscopic apparatus comprising, a calibration algorithm for Met-Hb and one or more than one calibration algorithm for the one or more than one Hb-based blood substitute;
ii) calculating a first concentration of the Met-Hb from the absorbance, by applying the calibration algorithm for Met-Hb to an order derivative of the absorbance, and calculating a second concentration of the one or more Hb-based blood substitute from the absorbance, by applying the one or more than one calibration algorithm for the Hb-based blood substitutes to an order derivative of the absorbance;

where, if the first concentration of the Met-Hb is greater than or equal to 3% of the second concentration of the one or more than one Hb-Based blood substitute, than this indicates degradation of the one or more than one Hb-based blood substitute.

Preferably, the sample is selected from the group consisting of, a whole blood sample obtained from a patient infused with one or more than one Hb-based blood substitutes, a serum sample obtained from a patient infused with one or more Hb-based blood substitutes, a plasma sample obtained from a patient infused with one or more Hb-based blood substitutes, and a stock Hb-based blood substitute.

The present invention pertains to either the method (D), (E) or (F) described above wherein in the step of measuring or determining (step i), the spectroscopic apparatus comprises:

a) one or more than one source of electromagnetic radiation (EMR) that produce a light path;

b) one or more than one photodetector in alignment with the light path;

c) a sample slot for receiving a sample vessel to be placed within the light path;

d) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

Additionally, the sample vessel may be selected from the group consisting of, a cuvette, a sample tab, a pipette tip, tubing, a labeled test tube, an unlabeled test tube, blood bag tubing, a transparent sample container, a translucent sample container, and a flow-through cuvette.

The present invention describes a method for measuring an analyte, for example, Total-Hb more accurately, by a spectroscopic method, and also for measuring at the same time, Met-Hb from natural Hb or Hb-based blood substitutes. An apparatus for use in analysing the analyte is also described.

This summary of the invention does not necessarily describe all features of the invention.

**BRIEF DESCRIPTION OF THE DRAWINGS**

These and other features of the invention will become more apparent from the following description in which reference is made to the appended drawings wherein:

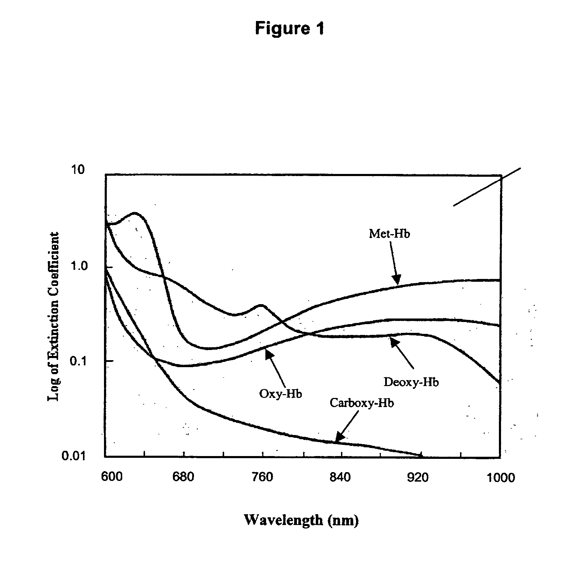

**Figure 1** shows a graphic representation of the absorbance spectra of four different hemoglobin species, as shown, in the wavelength range of 600 - -1000nm plotted on the x-axis, and log of extinction coefficient plotted on the y-axis.

**Figure 2** shows a graphic representation of the absorbance spectra of four different hemoglobin species, as shown, in the wavelength range of 500 - 700nm plotted on the x-axis, and absorbance of the same concentration of each specie (equivalent to extinction coefficient) on the y-axis.

**Figure 3** shows a graphic representation of the absorbance spectra of three different concentrations of total Hb, from the same pool. The total Hb was allowed to become partly oxidized to produce Met-Hb, which is also shown.

**Figure 4** shows various aspects of a sample tab that may be used in accordance with the present invention. A reflector may be positioned underneath the sample tab for use in reflection mode. Figure 4a illustrates oblique views of a sample tab and a sample slot in an spectroscopic apparatus. Figure 4b exhibits a side view of the sample tab inserted in the slot.

**Figure 5** shows various aspects of a sample tab used in accordance with the present invention. The sample tab is shown for use in transmission mode. **Figure 5a** illustrates oblique views of a sample tab and a slot. Figure 5b exhibits a side view of the sample tab inserted in the sample slot.

**Figure 6** shows more details of an apparatus of the present invention illustrated in **Figure 5. Figure 6a** exhibits a side view of a sample tab inserted in a sample slot. **Figure 6b** exhibits a front view of the sample tab inserted into the sample slot.

**Figure 7** shows various aspects of an alternate embodiment of a sample tab used in the present invention. **Figure 7a** illustrates an oblique view of the sample tab. **Figure 7b** exhibits a side view of the sample tab.

**Figure 8** shows a spectrometer **614** (with a cut-out view) used in the preferred embodiment. For simplicity, only two photodiodes are shown.

**DETAILED DESCRIPTION**

This invention relates to the field of spectroscopic measurements of analytes. More specifically, the invention relates to the method and apparatus used for Hemoglobin measurement and related substances.

The following description is of a preferred embodiment.

The present invention provides a spectroscopic apparatus. This apparatus may be used for determining the concentration or presence of a desired analyte within a sample, and may comprise:

a) a source of electromagnetic radiation (EMR) capable of producing wavelengths, for example, from about 300nm to about 2500nm, or any wavelength therebetween;

b) a first aperture located between the source of EMR and a sample slot to produce a light path therebetween;

c) the sample slot in the apparatus for receiving a sample vessel to be placed within the light path;

d) a second aperture located in the light path, between the sample slot and one or more than one photodetector, the one or more than one photodetector in operative association with the spectroscopic apparatus; and

e) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

The present invention also provides a method for measuring Corrected Total-Hemoglobin (Corr-Total-Hb) in a sample. This method may comprise:

i) developing a first primary calibration algorithm for one or more than one of Total-Hb, Oxy-Hemoglobin (Oxy-Hb), and "Total Hemoglobin minus Met-Hemoglobin" (Total-Hb minus Met-Hb), for predicting a concentration for one or more than one of the Total-Hb, Oxy-Hb, and "Total-Hb minus Met-Hb," in the sample, and producing a first value;

ii) deriving a second primary calibration algorithm for Met-Hb, for predicting a concentration of the Met-Hb in the sample and producing a second value; and

iii) measuring the sample using a spectroscopic apparatus to obtain the first and second values; and

iv) adding either:

- the second value to the first value to produce the Corrected Total-Hemoglobin; or

- terms of the first primary calibration algorithm for one of the one or more than one of Total-Hb, Oxy-Hb, and "Total-Hb minus Met-Hb," to terms of the second primary calibration algorithm to produce a single set of terms for a single calibration algorithm, which measures the Corrected Total-Hemoglobin.

There is also provided an alternate method for measuring Corrected Total-Hemoglobin (Corr-Total-Hb) in a sample. This alternate method may comprise:

i) providing a spectroscopic apparatus comprising one or more than one first primary calibration algorithm and a second primary calibration algorithm, the first primary calibration algorithm developed for one or more than one of Total-Hb, Oxy-Hemoglobin (Oxy-Hb), and "Total Hemoglobin minus Met-hemoglobin" (Total-Hb minus Met-Hb), for predicting a concentration for one or more than one of the Total-Hb, Oxy-Hb, and "Total-Hb minus Met-Hb," in the sample, thereby producing a first value, and the second primary calibration algorithm developed for Met-Hb, for predicting a concentration of the Met-Hb in the sample and producing a second value;

ii) measuring the sample using the spectroscopic apparatus to obtain the first and second values; and

iii) adding either:

- the second value to the first value to produce the Corrected Total-Hemoglobin; or

- terms of the first primary calibration algorithm for one of the one or more than one of Total-Hb, Oxy-Hb, and "Total-Hb minus Met-Hb," to terms of the second primary calibration algorithm to produce a single set of terms for a single calibration algorithm, which measures the Corrected Total-Hemoglobin.

The present invention also is directed to a method for flagging a predicted value for an indicator of hemolysis in a sample, or a predicted value for Total-Hb in a sample, for the presence of Met-Hb. This method may comprise:

i) developing a first primary calibration algorithm, using a spectroscopic apparatus, for one or more than one of Total-Hb, Oxy-Hb, or "Total-Hb minus Met-Hb," for predicting a first value for one or more than one of the Total-Hb, Oxy-Hb, or "Total-Hb minus Met-Hb," in the sample, wherein each of the Total-Hb, the Oxy-Hb, or the "Total-Hb minus Met-Hb," is used as an indicator of hemolysis in the sample;

ii) deriving a second primary calibration algorithm for Met-Hb, for predicting a second value for the Met-Hb in the sample; and

iii) flagging the first value if the second value exceeds a pre-determined value.

Also disclosed herein is a method of monitoring degradation or reversal of degradation of one or more than one Hb-based blood substitute in a sample. This method may comprise:

i) measuring the sample using a spectroscopic apparatus comprising a calibration algorithm for Met-Hb, at one or more than one wavelengths of a standard set of wavelengths, to obtain an absorbance;

ii) determining a first concentration of the Met-Hb from the absorbance, by applying the calibration algorithm, to an order derivative of the absorbance;

iii) determining a second concentration of the Met-Hb in the sample at a second time;

where degradation of the one or more than one blood substitute is indicated by an increase in the second concentration compared to the first concentration, and where reversal of degradation of the one or more than one blood substitute is indicated by a decrease in the second concentration when compared to the first concentration.

The present invention also provides for an alternate method of monitoring degradation of one or more Hb-based blood substitutes in a sample comprising:

i) determining a first concentration of Met-Hb, and a second concentration of the one or more than one Hb-based blood substitutes in the sample, by applying a first calibration algorithm for the Met-Hb, and a second calibration algorithm for the one or more than one Hb-based blood substitutes, to an order derivative of absorbance of the sample at one or more than one wavelengths of a standard set of wavelengths;

ii) calculating a proportion of the one or more than one Hb-based substitutes that is in the form of Met-Hb; and

iii) using the proportion of Met-Hb as a measurement of degradation of the one or more than one Hb-based blood substitutes to monitoring degradation of the one or more than one Hb-based blood substitutes. For example, if the first concentration of the Met-Hb is greater than or equal to 3% of the second concentration of the one or more than one Hb-Based blood substitute, than this indicates degradation of the one or more than one Hb-based blood substitute.

Technical terms used herein are defined below for clarification.

By "analyte" it is meant a substance being measured in a sample. Examples of samples within which analytes are to be measured include, but are not limited to, biological samples for example whole blood, serum, plasma, urine, synovial fluid and cerebrospinal fluid, sputum, lymphatic fluid, semen and feces, or non-biological samples selected from the group consisting of milk, cheese, cottage cheese, yogourt, ice cream, wine, and other beverages, semi-solid food and soft solid food.

By "absorbance" it is meant a reduction of light intensity caused by a sample. According to Beer's law, Absorbance = Log(1/Transmitted light), which applies to non-light-scattering samples. The measured parameter is the amount of light transmitted through a sample, and the transmitted light (or transmittance or transmission) is then converted to absorbance units. When a sample is light-scattering and Beer's law is applied, an apparatus cannot distinguish "true absorbance" from loss of light due to scattering, hence the term "apparent absorbance" should be used. It should be understood that when the term "absorbance" is used, it could mean either "true absorbance" or "apparent absorbance," or both, since it is not always obvious whether the sample is light-scattering or non-light-scattering. Although examples are given with respect to absorbance, it should be understood that absorbance can be replaced with Log (1/Reflectance), when reflectance (or reflection) is measured instead of transmittance, and reflectance measurement is within the scope of the present invention. It should be understood that the terms transmittance and transmission are sometimes used interchangeably. It should also be understood that the terms reflectance and reflection are sometimes used interchangeably.

By "actual absorbance" or "measured absorbance" it is meant the absorbance value, or absorbance measurement, or simply absorbance of a sample or calibrator material that is provided by the apparatus at one or more given wavelength(s) from a wavelength calibration table of the apparatus.

By "adjusted interpolated absorbance" it is meant the value of the interpolated absorbance after photometric correction is applied specifically to the interpolated absorbance.

By "blood bag tubing" it is meant the tubing connecting a first bag made of any suitable polymer or plastic that contains whole blood and a second bag made of any suitable polymer or plastic that may contain plasma obtained from the first bag. The tubing and bags may be made from transparent or translucent flexible polymer or plastic.

By "blood substitute" it is meant any substance that can be used instead of whole blood or red blood cells (RBC's) for blood transfusion. Some advantages of using a blood substitute instead of blood or red blood cells are as follows: blood substitutes are expected to be universally compatible with all blood types, therefore cross-matching will not be necessary; maximum storage time of blood is 42 days, whereas the blood substitutes could have a much longer shelf-life; the purification process of the blood substitute may include heat treatment, which could eliminate the threat of hazardous viruses.

Another type of blood substitute has been reported, which is characterized as a milky-white emulsion containing tiny beads of perfluorocarbons within a suitable surfactant. These "milky-white" blood substitutes may be referred to as "perfluorocarbon-like" blood substitutes. It should be understood that the term perfluorocarbon-like blood substitutes refers to all blood substitutes that are characterized as milky-white emulsions. Due to the beads contained with these blood substitutes, "perfluorocarbon-like" blood substitutes are characterized as comprising a component that scatters light.

By "interferents" it is meant an analyte whose presence in a sample, for example a serum or plasma sample, interferes with the determination of the presence, the quantification, or both, of another analyte within the sample.

By "calibration algorithm transfer" it is meant the process of transferring a calibration algorithm from a first apparatus to a second apparatus. For the convenience of transferring a calibration algorithm form a first apparatus to a second apparatus, it is preferred that a standard set of wavelengths are used. The process of calibration algorithm transfer is disclosed in US 6,651,015 (Samsoondar; which is incorporated herein by reference). The method used to calibrate a first apparatus, wherein the apparatus can be used to measure the concentration of at least one analyte, is referred to as primary calibration. Primary calibration is a complex process and is described herein under the title "Primary Calibration." Due to its complexity, performance of primary calibration on every apparatus is not practical or desirable.

The present invention provides a simple alternative that allows an apparatus, for example a second apparatus, to function as though it was calibrated by the process of primary calibration. The second apparatus need not be calibrated in the same way in which the first apparatus was calibrated, in that there is no need to conduct a primary calibration using the second apparatus. It is preferred that the first and second apparatus are similar, however, this is not always required, depending upon the accuracy or type of measurement required by using the second apparatus.

The present inventor has found that for a given analyte, a "primary calibration algorithm" developed using one or more "first apparatus" can be transferred onto a "second apparatus," and the second apparatus used directly following calibration algorithm transfer. Additionally, the transferred primary calibration on the second apparatus may be upgraded, if desired, using a small set of unique calibrator materials that are distinct from the primary calibration set.

By "data pre-processing" it is meant any mathematical manipulation of spectroscopic data, which can be used to facilitate measurement of an analyte on an apparatus, including a first, second, or both, apparatus. Examples of data pre-processing, which should not be considered limiting in any way are:

- calculation of absorbance of electromagnetic radiation (EMR) transmitted through or reflected from a sample;

- calculation of interpolated absorbances;

- smoothing of absorbances; calculation of a first and higher order derivative of absorbance;

- multiplicative scatter correction;

- data transformation; and

- photometric correction.

It should be understood that any one or more forms of data pre-processing can be used prior to development of a calibration algorithm, and any one or more forms of data pre-processing can be used on the data from a second apparatus, prior to applying the calibration algorithm for calculating the concentration of an analyte. A non-limiting example of smoothing includes averaging of data.

By "Data Transformation" it is meant any mathematical technique that can be applied to either spectroscopic data or analyte concentration data. Examples of data transformation, which should not be considered limiting in any way, include Fourier Transformation of spectroscopic data, and calculation of the log or anti-log of an analyte concentration. It should be understood that smoothing can also be considered as data transformation, for example when the Savitzky-Golay method (Savitzky and Golay 1964, Anal. Chem., 36:1627-1638) is used.

By "derivative of absorbance" it is meant an order derivative of the absorbance. A zero order derivative of absorbance is the measured absorbance. The first order derivative of absorbance at a particular wavelength is the slope of the absorbance spectrum at that wavelength; the second order derivative of absorbance at a particular wavelength is the slope of the first derivative absorbance spectrum at the wavelength. Higher order derivative (third, fourth etc.) of absorbance can similarly be obtained by taking the slope of the derivative absorbance spectrum of the order immediately below (second, third etc.) Methods of calculating a derivative of absorbance at a particular wavelength are well known by those skilled in the art.

Calculation of the first derivative of absorbance at a particular wavelength may consist in taking the difference in absorbances at the two wavelengths that encompass the wavelength of interest. Other methods of calculating derivative of absorbance may use the absorbances at several different wavelengths, where smoothing is an integral part of the derivative process. It should be understood that with a greater degree of smoothing, there is also a greater loss of signal details in the absorbance spectrum or the order derivative of the absorbance spectrum. The minimum number of wavelengths that may be used to calculate a derivative of absorbance is two wavelengths. Smoothing, data transformation, and calculation of order derivatives of absorbances are non-limiting examples of data pre-processing. Other forms of data pre-processing, as described above, may be performed either before or after calculation of an order derivative of absorbance, for example, but not limited to, multiplicative scatter correction.

By "first apparatus" it is meant an apparatus used to develop one or more than one primary calibration algorithm. One or more than one first apparatus may be used to develop a primary calibration algorithm.

By INTRALIPID™ (IL) it is meant a lipid emulsion that simulates naturally occurring chylomicrons in blood. IL is one example of such an emulsion. The major cause of turbidity in serum and plasma is fat particles, for example chylomicrons, therefore IL, or other lipid emulsions may be used to simulate turbidity in blood. The term "simulator of turbidity" is used to refer to the "analyte" measured to quantify turbidity.

By "indicator of hemolysis" it is meant any substance present within a red blood cell (RBC) and not present in the plasma that surrounds the RBC. An example of an indicator of hemolysis includes, but is not limited to, Total-Hb, Oxy-Hb or "Total-Hb minus Met Hb." A sample of known Oxy-Hb concentration where the Oxy-Hb fraction is about 95% or the Total-Hb, can be considered to have a Total-Hb concentration of the same value as the Oxy-Hb concentration. Similarly, a sample of known Total-Hb concentration that comprises about 95% Oxy-Hb, can be considered to have an Oxy-Hb concentration of the same value as the Total-Hb concentration. Acceptability of the approximation of Total-Hb or Oxy-Hb concentration, depends on the required accuracy of the predicted value of the Total-Hb or the Oxy-Hb.

By "mapping" it is meant a process of associating an interpolated absorbance value with a standard wavelength.

"Multiplicative scatter correction" (also known as multiplicative signal correction) is a mathematical technique that may be used to remove at least some of the light scattering effect in the spectroscopic data obtained from a sample set. The technique rotates each absorbance spectrum so that it fits as closely as possible to the mean spectrum. The technique is described in more details in: Martens, H and Naes, T (Multivariate Calibration, 1993, Published by John Wiley & Sons); and Osborne, B.G., Fearn, T & Hindle, P.H. (Practical NIR Spectroscopy with Applications in Food and Beverage Analysis, 1993, Published by Longman Scientific & Technical), both of which are incorporated herein by reference. It should be understood that the mean spectrum for a sample set can be obtained after combining one or more than one sample absorbance measurement obtained from one or more than one apparatus.

By "photometric correction" or "absorbance adjustment" it is meant an adjustment made to an absorbance of a sample tested on one apparatus to make it appear as if the sample was tested on another apparatus. The amount of photometric correction is determined by the slope ("m") and y-intercept ("c") of the linear regression equation of the form "y-mx+c," obtained from the absorbances obtained from a set of calibrators on both the first apparatus, and a second apparatus during the process of calibration algorithm transfer. The resulting absorbance after photometric correction is referred to as adjusted absorbance or corrected absorbance.

By "pixeldispersion" it is meant, the wavelengths encompassed by two adjacent pixels of a linear diode array, usually measured in nanometers (nm) per pixel. For example, if two lasers of 600 nm and 900 nm are used for wavelength calibration, and they are projected on pixel 20 and pixel 220 respectively, that means 300 nm (i.e., 900-600 nm) are encompassed by 200 pixels (i.e., 220-20 pixels). Therefore the pixeldispersion is calculated to be 1.5 nm per pixel (i.e., 300 nm divided by 200 pixels). Alternatively, a predetermined pixeldispersion may be used, in which case, only a single laser of known wavelength or narrow bandpass filter that provides EMR of a known wavelength, is required to assign a wavelength to a pixel.

By "primary calibration" it is meant a process used to develop a primary calibration algorithm for a first apparatus for an analyte or optionally for more than one first apparatus. Typically, the sample set used for primary calibration is relatively large, and the samples are natural or very close to natural samples. The primary calibration set should include all the variability expected in a sample, in order to develop robust calibration algorithm(s). Furthermore, one, or more than one sample of the primary calibration set could be measured on one or more than one first apparatus and combined, in order to develop a more robust calibration algorithm(s) that also includes inter-apparatus variability. Such a calibration algorithm would be developed using a combination of measurements obtained from one, or more than one, similar apparatus.

Any form of statistical data analysis and optionally any form of data pre-processing, for example but not limited to, smoothing, calculation of first and higher order derivative of absorbance, photometric correction, data transformation, interpolation of absorbance, or multiplicative scatter correction, may be used, depending on the required accuracy of the analyte prediction. For example, by including data from more than one first apparatus, a lower level of precision and hence a lower level of accuracy (poor precision translates into poor accuracy) may be obtained across many second apparatus. Such a type of primary calibration would be suitable if a simple yes/no answer to the presence of an analyte in a sample is all that is required, and is within the scope of this invention.

If desired, a small set of unique samples which are not part of the primary calibration set can be measured on a second apparatus, and the data combined with some or all of the original data from the primary calibration set, to develop one, or more than one, "upgraded primary calibration algorithm." Preferably, the small set of unique samples are similar to the samples of the primary calibration set. Zero order derivative of absorbance (also referred to as raw absorbance) or any order derivative of absorbance may be used in the calibration process with second order derivative of absorbance being preferred, and first order derivative of absorbance being more preferred.

By "primary calibration algorithm" it is meant a mathematical equation, for example, but not limited to a linear combination of the type $Y=A(x)+Bx_1+...+C$ where Y is the concentration of a given analyte, A, B and C are constants and $x$, $x_1$, ... are the order derivative of absorbance values at specified wavelengths (The right side of the equation consists of the summation of "terms" of the equation). However, non-linear equations are within the scope of the present invention (e.g. Equations 16 and 17, Example 5). The equation is preferably obtained by multiple linear regression of a sample set, but other statistical techniques for example but not limited to, simple linear regression, PLS (partial least squares regression) and PCA (principle component analysis) may also be used and are within the scope of this invention. The sample set used for primary calibration is relatively large (see above), and the samples are natural or very close to natural samples. The primary calibration set should include all the variability expected in a sample, in order to develop robust calibration algorithm(s). The term "calibration algorithm" when used and unless otherwise specified, means the primary calibration algorithm, or any modification of the primary calibration algorithm (for example an upgraded primary calibration algorithm), whereby the modification is for improvement in accuracy of predicted values of an analyte, or to facilitate use of the primary calibration algorithm on another apparatus that was not calibrated as the first apparatus.

When a primary calibration algorithm is installed on a spectroscopic apparatus used for determining the concentration of an analyte in a sample, the primary calibration algorithm is to be in operative association with the spectroscopic apparatus within which it is installed. As noted above, a primary calibration algorithm is typically developed using a first apparatus and transferred onto a second apparatus for use in the second apparatus. Furthermore, a primary calibration algorithm that is in operative association with the spectroscopic apparatus may be an upgraded primary calibration algorithm that was developed on a first apparatus, transferred to a second apparatus and upgraded after transfer, using for example, a small set of unique calibrators that were not part of the primary calibration set. However, it is to be understood that a primary calibration algorithm may be transferred from the first apparatus for use in a second apparatus and used directly, without any further modifications, or upgrading, of the primary calibration algorithm. A primary calibration algorithm that is in operative association with the spectroscopic apparatus may be installed on ROM, EPROM, EEPROM, microcontroller, microprocessor, internal or external memory device, for example but not limited to a disc, a CD, a memory stick, a flash memory card, or similar device, of the spectroscopic apparatus.

The term "calibration algorithm" when used and unless otherwise specified, means the primary calibration algorithm, or any modification of the primary calibration algorithm, whereby the modification is for improvement in accuracy of predicted values of an analyte, or to facilitate use of the primary calibration algorithm on another apparatus (e.g. a second apparatus) that was not calibrated as the first apparatus.

By "primary calibration set" it is meant the samples used for primary calibration.

By "primary calibration wavelength(s)" it is meant the wavelength(s) used in a primary calibration algorithm.

By "principal calibration wavelength" it is meant a wavelength of the primary calibration algorithm exhibiting a high correlation between an order derivative of absorbance, and the analyte concentration. The principal calibration wavelength may be different for the same analyte in different compositions. The primary calibration algorithm may optionally comprise one or more other wavelengths exhibiting low correlations between an order derivative of the absorbance and the analyte concentration. These other wavelengths are referred to as secondary calibration wavelengths. Secondary calibration wavelengths add robustness to the primary calibration algorithm especially in the presence of interferents that may have absorption bands overlapping that of the principal calibration wavelength(s) and therefore affect the correlation between the absorbance at the principal calibration wavelength and the analyte concentration.

A continuous spectral segment having a negative slope of from about 5 to about 400 nm or an amount there between, or from about 5 to about 200nm or an amount there between, that contains at least one principal calibration wavelength is referred to as a "principal calibration section." For development of primary calibration algorithm, any statistical technique may be used for example, which should not be considered limiting in any way, simple linear regression, multiple linear regression, and multivariate data analysis. Examples of multivariate data analysis, which should not be considered limiting in any way, are Principal Component Analysis (PCA), Principal Component Regression (PCR), Partial Least Squares regression (PLS), and Neural networks. It should be understood that when multivariate analysis is used to develop a primary calibration algorithm, the primary calibration algorithm could contain many wavelengths at which high correlations between an order derivative of absorbance at respective wavelengths and the analyte concentration is observed.

By "predicted value," it is meant a value of an analyte obtained when the primary calibration algorithm for the analyte is applied to an order derivative of absorbance of a sample. As indicated earlier, a primary calibration algorithm is an equation comprising, for example, a predicted value of the analyte as the dependant variable, and a summation of a constant and one or more other terms. Each of the other terms is the product of a constant and an independent variable (see Examples 1 to 7). The independent variable is the order derivative of absorbance of the sample at a specific wavelength. It is to be understood that the predicted value need not necessarily be reported as a discrete concentration value, but may also include semi-quantitative or qualitative (e.g Yes/No) values.

By "reference value" of an analyte, it is meant the value of the analyte assigned to a sample. A reference value is typically estimated by a method known within the art, which has a suitable level of accuracy. For example which is not to be considered limiting in any manner, known amounts of an analyte added to a sample can be used as the reference value, or, as in the case of an indicator of hemolysis, the indicator of hemolysis can be measured. In the case of an indicator of hemolysis, the preferred indicators are Total-Hb, Oxy-Hb and "Total-Hb minus Met-Hb".

The cyanmethemoglobin (cyanMet-Hb) method, which is well known to a person of skill in the art will measure all the Hb species present, i.e., Oxy-Hb, Deoxy-Hb, Carboxy-Hb and Met-Hb. Oxy-Hb can be measured by known reagentless spectroscopic methods, for example Harboe or Tietz (Harboe, M., 1959, A method of determination of hemoglobin in plasma by near ultraviolet spectrophotometry. Scand. J. Clin. Lab. Invest., pp. 66-70; Tietz Textbook of Clinical Chemistry, 3rd Ed, 1999, pp 1674-1676; which is incorporated herein by reference). The Hb species actually measured by the reagentless spectroscopic apparatus depends on both the reference method used to measure the analyte, and the substances included in the samples of the primary calibration set.

A sample of known Oxy-Hb concentration where the Oxy-Hb fraction is about 95% of the Total-Hb, can be considered to have a Total-Hb concentration of same value as the oxy-Hb concentration. Similarly, a sample of known Total-Hb concentration that comprises about 95% Oxy-Hb, can be considered to have an Oxy-Hb concentration of the same value as the Total-Hb concentration.

By "sample" or "samples" it is meant a biological or non-biological fluids, a biological or non-biological semi-solid, or a biological or non-biological solid exhibiting one or more properties that may be measured spectroscopically. A sample typically comprises one or more than one analytes. Examples of a sample include, but are not limited to, a calibrator, whole blood, serum, plasma, urine, synovial fluid, lymphatic fluid, sputum, feces, dairy products, beverages, a body part, for example but not limited to, a finger, arm, ear lobe, or a pharmaceutical tablet.

By "sample vessel" it is meant any transparent or translucent container capable of holding a sample to enable measurement of absorbance, reflectance, or both absorbance and reflectance of EMR from the sample. Examples of a sample vessel includes, but is not limited to, a sample tab, a pipette tip, tubing, a cuvette, a labeled test tube, an unlabeled test tube, blood bag tubing, a transparent sample container, and a translucent sample container. The sample vessel may be inserted within a sample slot of spectroscopic apparatus.

In the case of a cuvette, it should be understood that the cuvette could be designed as a flow-through cuvette, which requires that the sample be injected into the reuseable cuvette. However, a flow-through cuvette is not preferred due to the requirement of a wash system, but a flow-through cuvettte is still considered to be within the scope of the present invention. The sample vessel may optionally contain one or more reagents. In the case of a body part, a receptor is required instead of a sample vessel.

The present invention need not be limited to a reagentless system, and the use of one or more reagents in the sample vessel is regarded as an enhancement of a reagentless system. Lilja et al in US Pat. No. 4,088,448 describe a cuvette for sampling, with a cavity that is defined by two planar surfaces, which are placed at a predetermined distance from one another, wherein the cavity contains a reagent, and the sample is optionally drawn into the cavity by capillary force. It should be understood that the use of such cuvette or any similar cuvette is considered to be within the scope of the present invention.

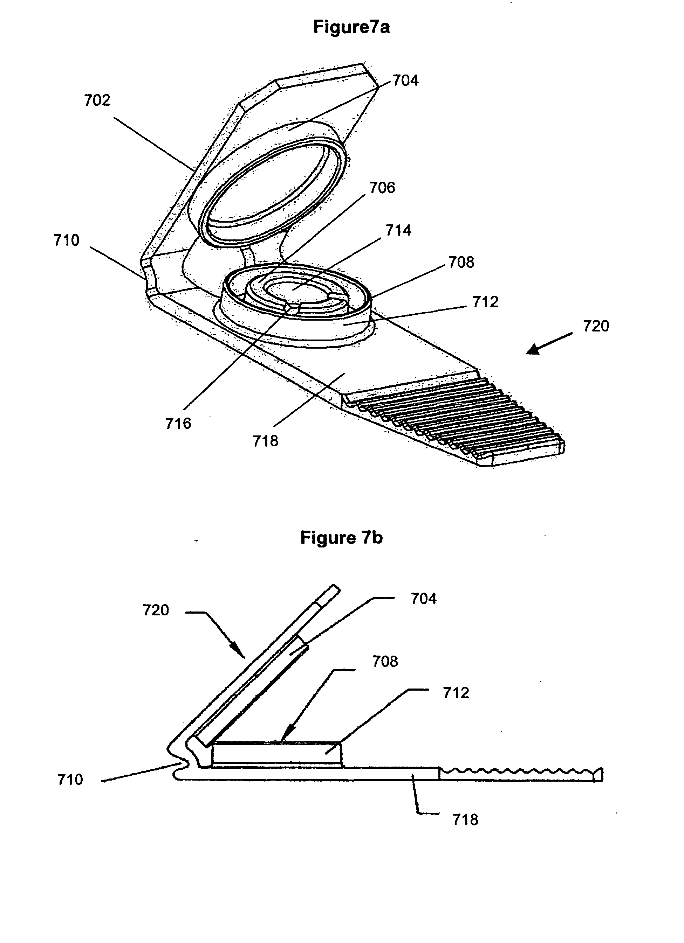

By "sample tab" it is meant a sample vessel comprising, a base plate having a top surface and a bottom surface, at least a portion of the base plate adapted to permit transmission of EMR therethrough, for example as shown in Figures 7a and 7b (720). A well (714) is disposed on the top surface of the base plate (718) for retaining a sample, for example a liquid sample, the well defined by a closed wall (706) extending above the top surface of the base plate, and a cover plate (702), preferably attached to the base plate, for example hingedly attached (e.g. 710) to the base plate, and moveable between an open and a closed position. The closed wall (706) of the sample tab may comprise one or more overflow openings (716), and surrounded by a containment wall (712) so that an overflow ring is defined between the closed wall and the containment wall.

At least a portion of the cover plate permits transmission of EMR therethrough, so that when the cover plate is in the closed position an optical path may be formed through the portion of the base plate that permits transmission of EMR, the well, and the portion of the cover plate that permits transmission of EMR. Alternatively, the sample tab may be configured so that EMR may be reflected off the opposite side of the sample tab, thereby doubling the direct pathlength through a sample present within the sample tab.

The cover plate may be attached to the base plate or may be separate. Further, the sample tab may comprise a locking member that associates with a corresponding mating member, thereby permitting the cover plate to be attached to the base plate. The locking member may comprise, but is not limited to, a circular ring capable of frictionally engaging an outer portion of a containment wall or one or more clips capable of frictionally engaging and attaching the cover plate to the base plate. Although a circular well and an overflow ring are shown in the example, it should be understood the well and overflow section may be of any shape. The locking members may be located on the base plate, cover plate or both the base plate, and the cover plate. Similarly, the associated mating member that receives the locking member may be located on the base plate, cover plate or both the base plate, and the cover plate.

The containment wall may comprise a sealing member on its upper surface (708). The sealing member may be an O-ring, or a pliable material integral with the containment wall. In a preferred embodiment of the present invention, the sample well contains one or more openings or grooves and an overflow ring for collecting excess sample, as closing the cover plate squeezes out excess sample. Preferably, the cover plate is attached to the tab so that the sample proximate the cover plate hinge makes contact with the cover plate first, and as the cover plate closes, excess sample is squeezed out through the two grooves and into the overflow ring. Other features and advantages of the present invention will become apparent from the following detailed description. It should be understood, however, that the detailed description and the specific examples while indicating preferred embodiments of the sample tab are given by way of illustration only. Various designs of sample tabs are described in US patent application 10/042,258; (Publication Number 2002-0110496 A1, Samsoondar; the contents of which are incorporated herein by reference).

By "smoothing" a curve, for example an absorbance spectrum, it is meant applying a mathematical function to the digital data to produce a "continuous spectrum" and thereby reduce the "noise" in the spectrum. Various degrees of smoothing may be applied to a curve. However, loss of analyte signal may be observed as a result of smoothing.

By "second apparatus" it is meant an apparatus that is allowed to function like a first apparatus, whereby the second apparatus need not be calibrated, or need not be calibrated in the same way in which the first apparatus was calibrated (i.e., by conducting a primary calibration). A unique set of samples distinct from the primary calibration set, may be measured on a second apparatus to develop an upgraded primary calibration algorithm, if desired.

By a "standard set of wavelengths" it is meant a set of wavelengths used by all apparatus in conjunction with the apparatus-specific wavelength calibration table, used to generate interpolated absorbances from the measured or actual absorbances. The actual absorbances of a sample tested on an apparatus are measured at wavelengths from the wavelength calibration table, and the actual absorbances may be interpolated and mapped onto the standard set of wavelengths. The primary calibration algorithm(s) is preferably applied to the mapped absorbances, but may be applied to the actual absorbances, particularly when the wavelength calibration table and the standard set of wavelengths are the same. Without wishing to be limiting in any manner, an example of a standard set of wavelengths includes 450nm to about 300nm, preferably, from about 450nm to 1100nm, in increments of 2 nm. However, other wavelength ranges and increments may be used as required, and as would be known by one of skill in the art. The range of the standard set of wavelengths may be derived from the wavelength calibration table, and the increment may be obtained by trial and error. The standard set of wavelengths may also be obtained by establishing a set of wavelengths common to the wavelength calibration tables of both first and second apparatus. Also, the standard set of wavelengths may be obtained by establishing a set of wavelengths that approximate the wavelengths of the wavelength calibration tables of both first and second apparatus.

By a "standard wavelength" it is meant a wavelength from the standard set of wavelengths.

By a "stock Hb-based blood substitute," it is meant a manufactured Hb-based blood substitute that is ready for use, for example, which should not be considered limiting in any way, for infusion into a patient. Hb-based blood substitutes may be used as a quality control material.

By "upgraded primary calibration algorithm" it is meant a calibration algorithm derived from a unique set of samples distinct from the primary calibration set, which are tested on a second apparatus, and the data combined with some or all of the original data from the primary calibration set, to develop one, or more than one, "upgraded primary calibration algorithm."

By "wavelength calibration" it is meant the calibration of a linear diode array detector, charged coupled detector, or any other like device, of a spectrometer, wherein wavelengths are assigned to each pixel in the linear diode array, or charged coupled detector.

By "wavelength calibration table" it is meant a table that provides the actual wavelength corresponding to or assigned to each pixel, which is a result of the wavelength Calibration.

APPARATUS

The apparatus of the present invention preferably comprises the following elements:

- one or more than one source of electromagnetic radiation (EMR) for illuminating a sample. The source providing EMR characterized as having one or more than one wavelength from about 300nm to about 2500nm, or any wavelength therebetween. Preferably, the wavelength is from about 450nm to about 1100nm, or any amount therebetween;

- one or more than one photodetector for measuring the amount of EMR transmitted through the sample, or reflected from the sample;

- an electronic board which optionally contains one or more than one of, an amplifier, an analog-to-digital converter, and a microcontroller, for processing the information received by the one or more photodetectors;

- a sample slot in the apparatus for locating the sample vessel; and

- one or more than one primary calibration algorithm in operative association with the spectroscopic apparatus, wherein the one or more than one calibration algorithm was developed completely on one or more than one other apparatus. One or more than one upgraded primary calibration algorithm (see "Calibration Algorithm Transfer," below) in operative association with the spectroscopic apparatus may also be used within an apparatus of the present invention.

The apparatus can operate in transmission mode or reflectance mode, as will be described in the examples.

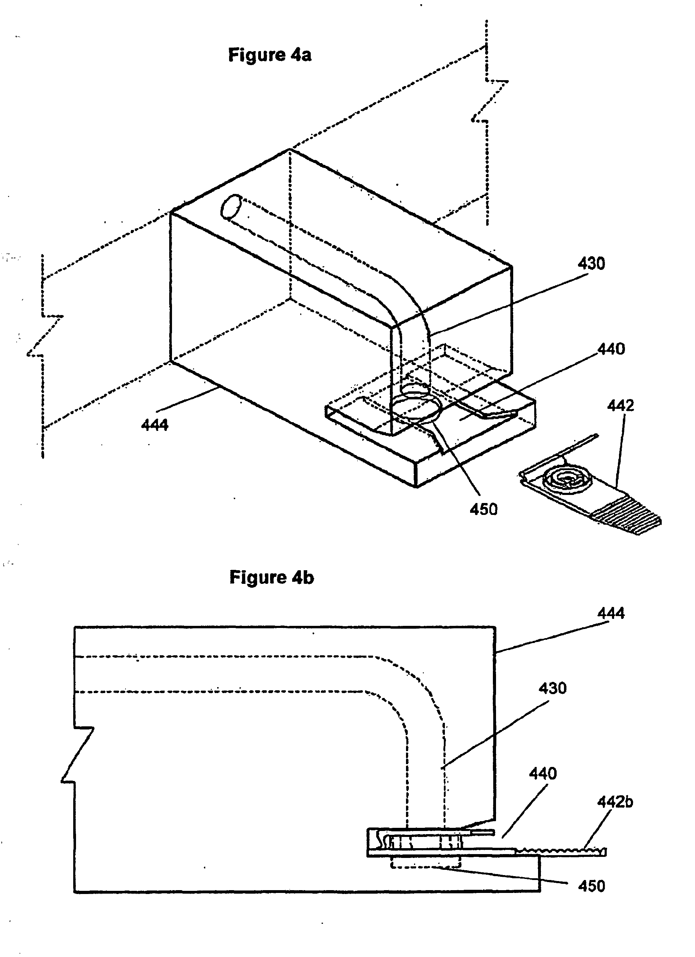

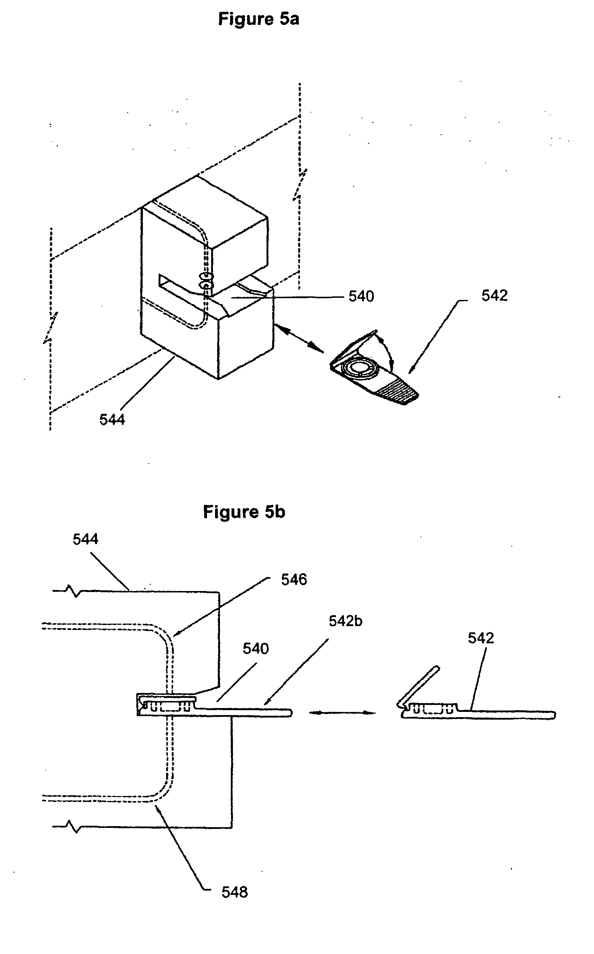

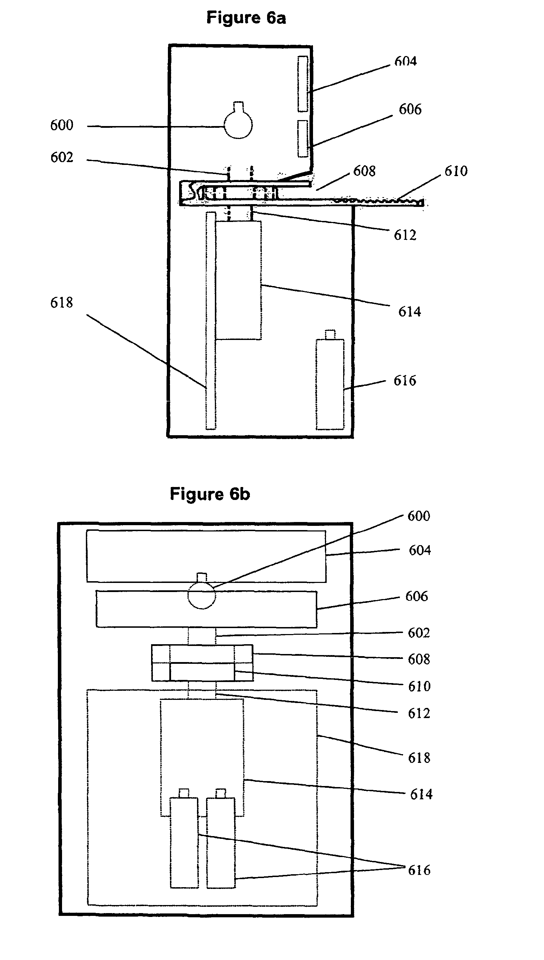

Referring now to Figures 4a and 4b, there is shown a sample interface (444) of a spectroscopic apparatus. For the purpose of clarity, the full spectroscopic apparatus is not shown in the figure. A bi-directional bundle of optical fibers 430 may be used, to transmit EMR to a sample place within a suitable holder, for example but not limited to a sample tab (442) and inserted within sample slot (440). Some of the fibers within the bundle 430 receive some of the EMR returning from the sample after EMR is reflected off a reflection member 450. The EMR collected after reflection is channeled to a spectrometer (discussed in more detail with respect to transmission mode and Figures 6a and 6b, below). Processing of EMR after reflection (reflection mode) is the same as the processing of EMR after transmission (transmission mode). Transmission mode is illustrated in Figures 5a, 5b, 6a and 6b. The sample interface (444) may be separate from the spectroscopic apparatus, and the incident and collection fibers carry the EMR signal to and from an external apparatus. However, as shown in Figures 6a and 6b, the sample interface may also be integral with the spectroscopic apparatus.

Figures 5a 5b show a sample interface (544) of a spectroscopic apparatus that may be used in transmittance mode. For the purpose of clarity, the full spectroscopic apparatus is not shown in the figure. A source of EMR may be provided to the sample (e.g. 542) via an incident optical fiber (548), and the EMR transmitted through the sample collected by a collection optical fiber (546). A sample may be placed within the sample slot (440) using any suitable sample holder, for example a sample tab (542, 542b). It should be understood that the incident optical fiber could be 546 and the collection optical fiber could be 548. As indicated above with respect to the reflectance mode, the sample interface (544) may be separate from the spectroscopic apparatus, and the incident and collection fibers carry the EMR signal to and from an external apparatus. However, the sample interface may also be integral with the spectroscopic apparatus (e.g. Figures 6a and 6b).

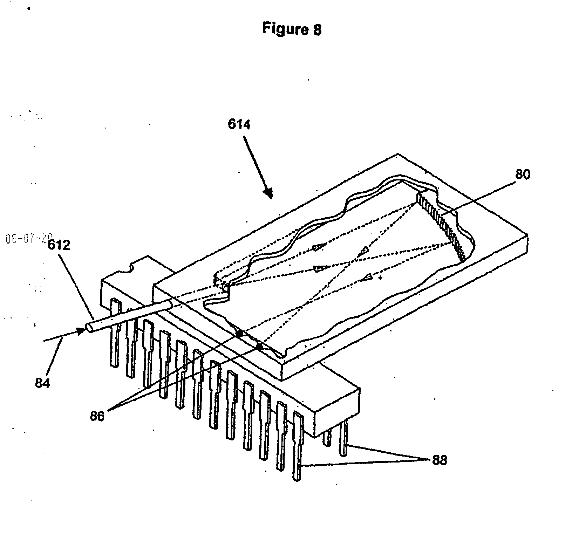

Referring now to Figure 6a & Figure 6b, there is shown a spectroscopic apparatus (620) comprising any desired source of EMR (600), for example a tungsten lamp. Attenuation of the EMR source may be required to prevent saturation of the detector within the spectrometer (614; detectors 86 are indicated in the spectrometer shown in Figure 8), and therefore an attenuating device may be placed between the source of EMR and the sample or detector. In the present example the attenuator is an aperture or channel (602) that can be of any appropriate diameter, but the channel could also be a fiber optic of any length or diameter, or other attenuating device, for example a filter or other device known to one of skill in the art that controls the amount of incident EMR reaching the sample slot (608).

A reference measurement can be taken before or after a sample measurement, or the reference measurement can be stored and reused any number of times. By inserting an opaque member in the sample slot (608), a dark current measurement can be made. By "dark current" it is meant the detector response when the detector is not exposed to EMR. Subtraction of a dark current measurement is optional, and no dark current measurement is required.

The EMR emerging from the sample slot (608) can enter the spectrometer (614), through channel 612. Channel 612, is shown as an aperture between the sample tab and the spectrometer, but the channel could be a fiber optic of any length, as shown in Figures 4 (430), % (546 or 548) or 8 (612).

The spectrometer, for example as shown in Figure 8, may comprise a diffraction grating 80. Either a transmission or reflection grating may be used. In the example shown in Figure 8, the diffraction grating 80 is a reflection grating. A grating is a dispersing element, which separates out the EMR component by wavelengths. In the preferred embodiment, the detector in spectrometer (614) is an array of photodiodes (e.g. 86 in Figure 8; for simplicity, only two diodes are shown in this figure), but the use of a single detector instead of an array of detectors may also be used. LED's may be used as a source of EMR, and with the use of LED's, a grating may not be required. For example, which should not be considered limiting in any way, a single detector could be used when the source of EMR (600) is one or more LED's.

The power source may be any suitable source, for example, which is not to be considered limiting, the power source in Figure 6 is shown as comprising two batteries (616). However, the apparatus may also be powered by an external power source, for example alternating current from a wall outlet.

The electronic signal received by the spectrometer is proportional to the time that the detector integrates the optical signal. The electronic signal may be amplified by analog electronic amplifiers (not shown) and converted to a digital signal by an analog-to-digital converter or ADC (also not shown).

Referring again to Figure 8, there is shown an example of a spectrometer that maybe used in accordance with the present invention. EMR emerging from the sample (84) impinges upon a reflection grating (80), and is dispersed into its component wavelengths. The dispersed EMR then impinges upon an array of diodes (e.g. 86), so each diode represents a pixel. The array has a known pixel dispersion, which would allow the assignment of wavelengths for each pixel. The array of pixels represents a range of wavelengths, for example the wavelength range may be about 450 nanometers to about 800 nanometers, with a pixel dispersion of about 3 nanometers per pixel. An example of a suitable spectrometer is produced by MicroParts, Germany, and contains 256 diodes. For simplicity, only two diodes (86) are shown in Figure 8. It should be understood that any number of pixels are within the scope of the present invention. Wavelength calibration (and a Standard Set of Wavelengths) of spectrometers is discussed in detail in US Pat. No. 6,651,015 (Samsoondar; which is incorporated herein by reference. The use of any spectrometer is considered to be within the scope of the present invention.

Also shown in Figure 8, is output from the diode array (88) that may be coupled to the electronic board (618 shown in Figures 6a and 6b). The electronic board (618) may also comprise an amplifier, an analog-to-digital converter, and a microcontroller, although these elements are not shown in Figure 6a and 6b.

As shown in Figures 6a and 6b, a sample tab (610) may be inserted in sample slot (608). Commands can be executed from a keyboard or keypad (606), and data, for example results, which should not be considered limiting in any way, may be displayed on a monitor or screen (604). It should be understood that the use of one or more switches, buttons, or keys are preferred to a keyboard or keypad, for a hand-held apparatus, and all are considered to be within the scope of the present invention. It should also be understood that use of a host computer is also considered to be within the scope of the present invention. Communication ports, which are not shown, are optional.

Appropriate shielding of the sample slot and detectors from room light may also be desired, but the extent of shielding depends on the analyte or parameter measured, and the use of dark current measurement. It should be understood that the apparatus could be oriented on any side, particularly with the top and bottom switched, i.e., with the source of EMR shown below the sample, instead of above as is Figures 6a and Figure 6b.

Absorbance is calculated by the microcontroller, which is installed (but not shown) on electronic board 618 as:

$$\text{Absorbance}_i = \log\{(RL_i - RD_i) / (SL_i - SD_i)\} + \log(ITS / ITR)$$

where:

Absorbance$_i$ = Absorbance at pixel i;

RL$_i$ is Reference Light$_i$ = Reference pixel i readings;

RD$_i$ is Reference Dark$_i$ = Reference pixel i readings;

Sl$_i$ is Sample Light$_i$ = Sample pixel i readings;

Sd$_i$ is Sample Dark$_i$ = Sample pixel i readings;

ITS = Integration time for sample measurement;

ITR= Integration time for reference measurement; and

i = the particular pixel (wavelength) in the array of detectors

The method of the present invention requires that one or more than one calibration algorithm for one or more analytes is installed in the spectroscopic apparatus, for example which is not to be considered limiting, the one or more than one calibration algorithm may be installed within the microcontroller which is integrated in the electronic board (618). However, one or more than one calibration algorithm could be installed in any form of non-volatile memory, for example, which should not be considered limiting in any way, ROM, EPROM, EEPROM (electronically erasable programmable read only memory), CD, diskette, or memory card.

The apparatus may comprise a sample slot (e.g. 540, Figure 5) for receiving a sample vessel (e.g. 542, Figure 5), for testing. By "sample slot" it is meant an opening through which the sample vessel is to be placed, or a groove or channel or slit into which the sample vessel fits. It should be understood that the slot could be oriented in any direction, but it is shown in Figures 5 and 6 as a horizontal slot, such that the EMR travels in the vertical direction. Alternate configurations include spectroscopic apparatus comprising a vertical sample slot, for receiving a sample vessel, for example a cuvette. In this configuration, the EMR passes though the sample in the horizontal direction.

As shown in Figures 5 and 6, the sample slot may be adapted to allow EMR to enter either a top side of the slot housing the sample vessel, and the transmitted EMR collected at the bottom side of the slot, or visa versa, with the incident EMR entering the bottom side of the slot, and exiting from the top side. The slot may also be adapted to allow EMR to enter the top side of the slot housing the sample vessel, where the transmitted EMR is reflected off a reflective surface or reflective member (e.g. 450; Figures 4a and 4b) located at either the bottom side of the slot. It should be understood that the transmitted EMR could be reflected off a reflective surface located on the side of the sample vessel at or near the back side of the slot, and the reflected EMR is collected at the top side of the slot.

The sample vessel may optionally contain one or more reagents, and the sample vessel may be any suitable vessel, including a cuvette or a sample tab, that may optionally contain one or more reagents.

## SAMPLE TAB

A non-limiting example of a sample vessel is a reagentless sample tab. The sample slot is designed to accept the sample tab in any suitable direction, for example a horizontal direction. A horizontal direction may be preferred when the sample is whole blood, since when whole blood is allowed to settle red blood cells tend to precipitate. In this case, in order for the red blood cells to remain in the path of the EMR, the EMR should travel in the vertical direction. However, any configuration of the sample slot is considered to be within the scope of the present invention. The sample vessel may also be a cuvette designed to draw in a sample by capillary action, and may optionally contain one or more reagents.

The sample tab may comprise a base plate with a sample well and a cover, wherein at least a portion of the base plate and at least a portion of the cover, is adapted to permit transmission of EMR therethrough. Alternatively, the sample tab may comprise a base plate with a sample well and a cover, wherein at least a portion of the base plate is adapted to permit transmission of EMR through the sample, and at least a portion of the cover is adapted to reflect EMR emerging from the sample, and wherein the reflected EMR is allowed to traverse the sample before leaving the sample tab at the base plate, or wherein at least a portion of the cover is adapted to permit transmission ofEMR through the sample, and at least a portion of the base plate is adapted to reflect EMR emerging from the sample, and wherein the reflected EMR is allowed to traverse the sample before leaving the sample tab at the cover. According to an aspect of the present invention, there is provided a sample tab for retaining a sample for testing. It should be understood that the sample tab is used as an example of a sample vessel, and should not be considered limiting in any way.

In use, a sample is retained in the well between the base plate and the cover plate of the sample tab so that electromagnetic radiation may pass through the base plate, through a sample in the well, and the cover plate. However, it is within the scope of the present invention that the radiation beam may travel though the sample, and be reflected off either the base plate or cover plate thereby doubling the path length of the radiation beam. By doubling the path length, a reduced volume of sample may be used during analysis. Either the base plate or the cover plate may have a reflective surface, or may be made of, reflective material. As an alternative, the sample tab may be made out of a transparent or translucent material, and still used in reflection mode as shown in Figures 4a and 4b. In this case the reflecting member (450) placed below the sample slot (440) may comprise for example but not limited to, a ceramic coating, barium sulfate, SPECTRALON™, SPECTRAFLECT™, or DURAFLECT.™

Referring now to Figures 7a and 7b, there is shown an aspect of an embodiment of the sample tab, which should not be considered limiting in any way. Sample tab (720) comprises base plate (718), cover plate (702) and sample well (714) defined by closed wall (706). Sample well (714) may be of any volume required, for example, but not limited to, a size sufficient to allow a drop of blood to fill the well, preferably with some excess. For example, which is not to be considered limiting, the well may be circular, as shown in Figure 7a and 7b, and comprises dimensions of about 4 mm in diameter and about 2 mm in depth.

Overflow openings or grooves (716) in closed wall (706) allow excess sample to flow out of sample well (714) when cover plate (702) is closed over sample well (714) and base plate (718). A second wall, such as, but not limited to, a containment wall (712) may be employed to retain the sample that overflows sample well (714), into an overflow ring (circular groove between wall 706 and wall 712) to prevent leakage of fluid from the sample tab, while permitting a sample of sufficient volume to fill the well. In this regard, the vertical height of containment wall (712) is less than or equal to the height of closed wall (706) defining sample well (714). More preferably it is equal to the height of closed wall (706) defining sample well (714). Cover plate (702) is preferably attached to base plate (718) by a hinge (710) or other suitable attachment means known in the art. However, a non-hinged cover plate may also be used, where the cover plate may be snapped on to the base plate.

The sample tab may be manufactured from any suitable material known in the art, for example, but not limited to, a transparent, translucent material, such as glass, plastic or a combination thereof, or a reflective material in parts. If the base plate and cover plate are transparent or translucent, then it is preferred that the base plate, and cover plate comprise a transparent or translucent plastic, such as but not limited to polypropylene, polycarbonate, polyethylene, or polystyrene, however, a glass plate may also be used. If either of the base plate or cover plate is reflective, then a reflective material, for example but not limited to a ceramic coating, barium sulfate, SPECTRALON™, SPECTRAFLECT™, or DURAFLECT™ may be used for one of the base or cover plates.

Optionally, the sample tab may comprise a locking member to lock cover plate (702) to the base plate (718). The locking member may comprise a portion of the cover plate, base plate or both. Further, the locking member may reversibly or irreversibly lock the cover to the base plate. Any locking member known in the art may be employed with the sample tab of the present invention, for example, but not limited to those as shown in US Patent Application No. 10/042,258 (Publication Number 2002-0110496 AI; Samsoondar; the contents of which are incorporated by reference). The use of a containment wall ensures that the sample is retained within the sample tab and reduces contamination between samples. Furthermore, by locking the cover plate of the sample tab in a closed position, the sample tab may be readily disposed of after use without sample leakage, or the sample tab may be used in a vertical position, for example within a cuvette holder adapted for use within spectroscopic apparatus.

Also shown is a locking member (704) which permits cover plate (702) to be fastened to base plate (718). In this example, the locking member (704) comprises a circular ring, capable of frictionally engaging the containment wall (712), thereby reversibly attaching cover plate (702) to base plate (718), preventing the escape of a sample from the sample tab.

When the cover plate is closed over the well, and attached to the base plate, it is preferred that the top surface (708) of the containment wall (712) seals against the lower surface of the cover slip. However, the locking member may also be used to help seal the sample within the sample tab should any leakage occur past the containment wall.

According to another aspect of the sample tab, the absorbance can be calculated from reflectance instead of transmittance. In the case of reflectance, either the base plate or the cover plate may have a reflective surface or may be made of reflective material. Such a reflective surface or material could include any suitable reflective coating, for example, but not limited to, a ceramic coating, barium sulfate, SPECTRALON™, SPECTRAFLECT™, or DURAFLECT™.

## WAVELENGTH CALIBRATION

Spectrometers should be calibrated, if wavelengths are used in the calibration algorithms, instead of pixel numbers. In order to facilitate calibration algorithm transfer, wavelength calibration of the spectrometer is required. Several methods of wavelength calibration are given below as example only, and should not be considered limiting in any way:

Method 1:

A laser of known wavelength or EMR transmitted through a band-pass filter of know wavelength, is projected onto any pixel in a linear diode array. It should be understood that the EMR should not be restricted to a laser or a band-pass filter, and other sources of monochromatic EMR may be used. It should also be understood that the EMR could impinge upon more that one pixel, and that the relative position of peak intensity of the EMR may be determined mathematically by processes known to those skilled in the art. Further, the peak intensity may be positioned between any two pixels. The targeted pixel is preferably towards one end of the spectrum. A second laser of known wavelength or EMR transmitted through a second band-pass filter of known wavelength that is preferably projected towards the other end of the spectrum may be used and the pixel on which the beam is projected onto is identified. Since the number of pixels is known, one can determine the pixeldispersion. With the two known wavelengths and their corresponding pixels, and the pixeldispersion, one can generate a wavelength calibration table i.e., a table providing the discrete wavelength that is assigned to each pixel in the linear diode array.

The absorbances at the wavelengths from the wavelength calibration table from one or more apparatus, can subsequently be interpolated and mapped unto a standard set of wavelengths. The absorbances at the two actual wavelengths that are on either side of the standard wavelength may be interpolated to produce an absorbance at a standard wavelength. This process may be repeated for each standard wavelength. This is, the preferred method for making the wavelengths provided by different apparatus, appear similar. Photometric accuracy depends in part on wavelength accuracy, and the prediction accuracy for an analyte concentration depends upon the photometric accuracy of the apparatus. In this respect, a qualitative method for an analyte where a yes/no answer is all that is desired does not require the same level of wavelength accuracy as a quantitative method for the same analyte. Futhermore, the calibration algorithm can be developed with more robustness by including data from one or more primary calibrators, measured on the first apparatus and one or more similar apparatus.

In this method of wavelength calibration, the first wavelength does not have to be projected upon the same pixel in the linear diode array of each apparatus, since the absorbances could be interpolated and mapped unto a standard set of wavelengths. The wavelength of a second laser or second band-pass filter is preferably chosen so that the beam of EMR is projected towards the other end of the linear diode array. It is preferred that the laser or band-pass filter be selected so that the beam of EMR is not projected too close to the end pixels in the linear diode array, if the resulting absorbances at the end pixels are noisy. It is also preferred that a bandpass filter is a narrow bandpass filter.

Method 2:

A second method to generate a wavelength calibration table is to project the first beam onto the same pixel of each linear diode array. When this method is used to generate a wavelength calibration table, the pixeldispersion is predetermined using two beams of different wavelengths, as described above. The pixeldispersion may be determined from a single spectrometer, but preferably the average value should be obtained from more than one like spectrometer. When the same pixeldispersion is used by each apparatus and the first beam is projected onto the same pixel number within each like linear diode array, the wavelength calibration table for each apparatus would be the same, and hence the wavelength calibration table may be used as the standard set of wavelengths. Consequently interpolation and mapping of absorbances to a standard set of wavelengths would automatically be eliminated. A second beam may be used to validate wavelength accuracy.

Method 3:

A third method to generate a wavelength calibration table is like the second method except that the first beam may be projected onto any pixel of the linear diode array. When the pixel number that the first beam is projected onto, is different in different apparatus, the pixel numbers assigned to a specific wavelength in the wavelength calibration table of the different apparatus will differ. In this case, software may be used to produce a standard set of wavelengths as follows:

(i) Establish a set of wavelengths common to the wavelength calibration table of the different apparatus.

(ii) Select a range of wavelengths of the standard set of wavelengths, the range of wavelengths having wavelengths belonging to the standard set of wavelength.

It should be understood that the wavelength calibration table obtained from different apparatus as described in above third method may be such that a pixel number from different apparatus may not be assigned the same wavelength. It should also be understood that the first pixel may be an approximation to a pixel number and also the first pixels from different apparatus may be approximated to be the same pixel, and that the approximations tolerated depends on the prediction accuracy required for the primary calibration algorithms. In other words, the identification of the first pixel may be incorrect. An incorrect identification can be tolerated provided that the incorrectly identified pixel is within less than or equal to about +/-N pixel, where N is the number of pixels that encompass a range of wavelength. Fore example, if the pixel dispersion is 2 nm and if the tolerated error is +/-10 nm, then the incorrectly identified pixel must be no more than 5 pixels away on either side of the actual pixel on which the beam impinged. Different levels of error may be tolerated typically, but not limited to +/-2 nm to +/-20 nm and more preferably from +/-2 nm to +/-10 nm. Selection of a wavelength calibration method depends on the required prediction accuracy of the primary calibration algorithms.

**CALIBRATION ALGORITHM TRANSFER**

Another aspect of the present invention is calibration algorithm transfer. One or more than one calibration algorithm in operative association with the spectroscopic apparatus, for example, installed in the microcontroller in the electronics board (618) shown in Figures 6a and 6b is used for determining the presence or concentration of one or more than one analyte in a sample. It should be understood that the one or more than one calibration algorithm could be installed in any form of non-volatile memory, for example, which should not be considered limiting in any way, ROM, EPROM, EEPROM (electronically erasable programmable read only memory), CD, diskette, or memory card. The one or more calibration algorithms were previously developed on one or more first apparatus by the process of primary calibration, and the one or more calibration algorithms were transferred to other apparatus, referred to as second apparatus. Calibration algorithm transfer is discussed in US 6,651,015 (Samsoondar; which is incorporated herein by reference).

In the preferred embodiment, a primary calibration algorithm is developed completely on one or more than one other apparatus, and simply installed in the apparatus of the present invention; no adjustment of the constants or coefficients of the primary calibration algorithm is made in the preferred embodiment. However, it should be understood that a calibration algorithm could also be derived using a set of unique samples distinct from those used in the primary calibration set, which are tested on a second apparatus, and the data combined with some or all of the original data from the primary calibration set, to develop one, or more than one, "upgraded primary calibration algorithm." It should be understood that the use of one or more upgraded primary calibration algorithm is also considered to be within the scope of the present invention. Upgraded primary calibration algorithms are also discussed in US 6,651,015 (Samsoondar; which are incorporated herein by reference).

For greater accuracy of a predicted value of an analyte concentration, absorbances that are measured at actual wavelengths of the second apparatus, could be mapped to a standard wavelength of a set of standard wavelengths, by interpolating absorbances at actual wavelengths that encompass the standard wavelength. Mapping of absorbances and interpolation of absorbances are also discussed in US 6,651,015 (Samsoondar; the contents of which are incorporated herein by reference).

After calibration algorithm transfer, "photometric correction" or absorbance correction could also be performed, depending on the required accuracy of the analyte tested for. Photometric correction or absorbance correction is also discussed in US 6,651,015 (Samsoondar; the contents of which are incorporated herein by reference).

It should be understood that the terms spectrometer and spectrophotometer are sometimes used interchangeably, and the inventor does not make any distinction between the two terms.

## HEMOGLOBIN IN BODY FLUIDS

The accuracy of measurement ofHb as an indicator ofhemolysis, depends upon several factors, for example, which is not to be considered limiting:

1) The Hb species selected as the indicator of hemolysis;

2) The constituents of each sample in the primary calibration set used to develop the primary calibration algorithm; and

3) The Hb species included in the reference value for the indicator of hemolysis.

US 6,268,910 B1, US 5,846,492, WO-98/39634, and WO-97/47972 describe calibration algorithms for Hb, wherein Hb is used as an indicator of hemolysis. However, none of these documents indicate the Hb species used as an indicator of hemolysis, nor is there any suggestion that Total-Hb is used as the indicator of hemolysis.

It should be appreciated by those of skill in the art, that although a primary calibration algorithm is developed for a particular analyte using accurate estimates of the reference values for the analyte, other analytes or substances that are present in a sample may introduce errors in the predicted values for the analyte. This applies particularly to the predicted values of an indicator of hemolysis, where the Hb could exist as several Hb species, and these Hb species need to be accounted for in the primary calibration algorithm. For example, the indicator of hemolysis could be Total-Hb, and the reference measurement made using standard methods, for example but not limited to, the cyanMet-Hb reference method for Total-Hb measurement (Tietz Textbook of Clinical Chemistry, 3$^{nd}$ Ed, 1999, p1673-1674). If the Total-Hb present in the primary calibration samples is not comprised of a suitable variation of the Hb species, the Total-Hb predicted value for a sample with a high proportion of Met-Hb, could be underestimated significantly.

The required accuracy of measurement of the indicator of hemolysis depends on the application of the indicator of hemolysis. Any substance present within a red blood cell (RBC) and not present in the plasma that surrounds the RBC, can be used as an indicator of hemolysis, as hemolysis liberates substances contained within the RBC's into the plasma or serum. Hb is an example of a substance contained inside the RBC's, and is only present in serum and plasma if hemolysis has occurred.

Hemolysis can occur *in vitro*, for example if the sample was handled roughly, or hemolysis can occur *in vivo*, for example in patients with fragile RBC membrane or in patients with prosthetic heart valves. Therefore, for accurately measuring an indicator of hemolysis it is desirable to determine:

1) the full extent of a combination of *in vivo* and *in vitro* hemolysis;

2) the true level of hemolysis, for example to understand by how much the concentration of a substance like potassium can become artificially elevated in serum or plasma, due to *in vitro* hemolysis (potassium is another example of a substance released from hemolyzed RBC's, as its concentration within the RBC's is about 25 times that of plasma); and

3) the increase in absorbance of the serum or plasma due to the release of hemoglobin, in an effort to understand how and to what extent the artificially increased absorbance due to Hb, affects spectroscopic assays for other analytes.

Total Hb is a sensitive indicator of hemolysis, and provides a good estimate of the extent of hemolysis. The composition of normal Hb in arterial blood is about 95% oxy-Hb, about 1% Met-Hb, about 2% carboxy-Hb, and about 2% deoxy-Hb, measured in an arterial blood sample by CO-oximetry. The art of CO-oximetry is well known and deals with the measurement of Hemoglobin species in whole blood: Oxy-Hb, Deoxy-Hb (or reduced-Hb), Met-Hb, and Carboxy-Hb. The proportion of the Hb species seen in most serum and plasma samples with hemolysis, is similar to that described for arterial blood, even though the serum and plasma is usually obtained from a venous blood sample. Although the percentage of Oxy-Hb of Total-Hb, called the Hb oxygen saturation, is usually much higher in an arterial blood sample, compared to that of a venous blood sample (because of the increase in Deoxy-Hb in venous blood), the increase level of Oxy-Hb in a venous sample (serum or plasma) is due to exposure of the sample to air, which contains 20% oxygen (i.e., a partial pressure of oxygen of 152mm Hg, 20% of 760mm Hg). Therefore, Oxy-Hb is another sensitive indicator of hemolysis, especially in blood samples with normal Hb species.

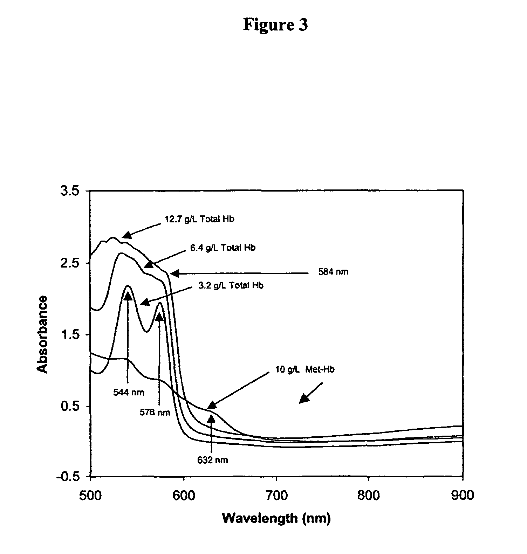

An increase in Met-Hb within a sample is shown in Figure 3, but the fraction of the Total-Hb that is in the Met-Hb form is unknown. The Met-Hb shown in Figure 3 was created by spontaneous oxidation of Hb. The blood donor used to provide the hemolysate with absorbance spectra shown in both Figure 3 is the same, and the absorbance spectra of the fresh hemolysate, made on different days, were indistinguishable. Although this discussion is more directed to hemolysis in serum and plasma, the same discussion could be applied to any body fluids, including whole blood.

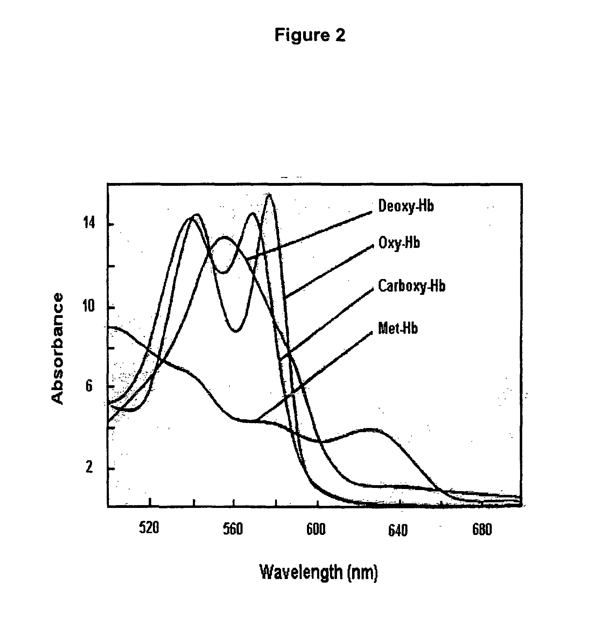

The absorbance spectra for Oxy-Hb, Deoxy-Hb and Carboxy-Hb are very similar in the region from about 576nm to 700nm (particularly from about 590nm to aabout 610nm, as shown in Figure 2), compared with absorbance of Met-Hb, (which is much lower) in the same wavelength region. Met-Hb also exhibits a characteristic absorbance peak at about 632nm. Therefore, if a calibration algorithm for Total-Hb is developed, for example, using reference values that are estimates of Total-Hb, comprising about 95% Oxy-Hb, large quantities of Deoxy-Hb and Carboxy-Hb in a sample would be included in the measurement of Total-Hb. However, the absorbance of Met-Hb is low in the 576nm to 700nm region, which could result in a significant underestimation of Total-Hb compared to the reference measurement of the Total-Hb. In this case, the predicted values derived from the calibration for Total-Hb as the indicator of hemolysis, would be more reflective of the "Total-Hb minus Met-Hb." In this example, the indicator of hemolysis may be more appropriately called, "Total-Hb minus Met-Hb."

In the example where the Oxy-Hb is about 95% of all the Hb species, the reference values of Oxy-Hb can be used as an estimate of Total-Hb. A sample of known Oxy-Hb concentration where the Oxy-Hb fraction is about 95% or the Total-Hb, can be considered to have a total Hb concentration of same value as the Oxy-Hb concentration. Similarly, a sample of known Total-Hb concentration that comprises about 95% Oxy-Hb, can be considered to have an Oxy-Hb concentration of the same value as the Total-Hb concentration. The predicted values of Oxy-Hb will not be significantly affected by Met-Hb, if affected at all, but the predicted values of Oxy-Hb will not be a reliable estimate of hemolysis, since most of the Met-Hb will not be measured.

Although the method of measuring Hb discussed above is with respect to contamination of a body fluid with Hb, or hemolysis in plasma and serum, it should be understood that measurement of Hb in whole blood is considered to be within the scope of the present invention. The only difference between Hb in whole blood and Hb in serum or plasma is the Hb concentration and the light scattering effect of RBC's. INTRALIPID particles may be added to some samples in the primary calibration sets for Hb in serum or plasma, to increase the light scattering effect typically associated with RBC's.

Therefore, an aspect of one of the methods of the present invention is to overcome the underestimation of Total-Hb in the presence of large quantities of Met-Hb as follows:

Method 1: Add Met-Hb to the primary calibration set, and include the Met-Hb in the reference values of Total-Hb for the development of a calibration algorithm; or

Method 2: Add Met-Hb in the primary calibration set, and do not include the Met-Hb in the reference value for Hb during development of the primary calibration algorithm.

In Method 1, the calibration algorithm for Total-Hb could partly include Met-Hb in the predicted Total-Hb results.

In Method 2, the calibration algorithm would predict "Total-Hb minus Met-Hb," and any Met-Hb in a sample would be ignored

Referring again to Method 2, a separate primary calibration algorithm may be developed for Met-Hb for determination of Met-Hb in a sample, to flag samples with Met-Hb that exceed a predetermined value, or the predicted Met-Hb could be added to the "Total-Hb minus Met-Hb" described above, for a determination of Total-Hb. Method 2 defined above, is an accurate method of obtaining Total-Hb in the presence of Met-Hb.

A primary calibration algorithm for "Total-Hb minus Met-Hb" may be developed using samples in the primary calibration set that contain various amounts of Oxy-Hb, Deoxy-Hb, Carboxy-Hb, and Met-Hb. It is preferred if the amounts of Oxy-Hb, Deoxy-Hb, and Carboxy-Hb, are summed to produce the concentration of Total-Hb (which is actually "Total-Hb minus Met-Hb") in the reference values. The name of the substance used as an indicator of hemolysis is usually the same as the substance or substances included in the reference values. However, it should be understood that the actual substance or substances included in the reference values depend on the composition of the primary calibrators.

It should be understood that Method 1 can be used if the accuracy of the estimated Total-Hb obtained using Method 1, is acceptable for the particular application.

In an aspect of the present invention, the terms of the primary calibration algorithm for "Total-Hb minus Met-Hb," and the terms of the primary calibration algorithm for Met-Hb are added to produce a set of terms for a single calibration algorithm, which predicts Corrected Total-Hb (Corr-Total-Hb).

In yet another aspect of the present invention, the indicator of hemolysis is Oxy-Hb, and a Corr-Total-Hb value can be obtained by adding the predicted values for Oxy-Hb and Met-Hb. To those skilled in the art, it will be understood that a significant proportion of Deoxy-Hb and/or Carboxy-Hb, ifpresent in a sample, could be measured as Oxy-Hb.

As noted above, about 95% of the Hb in a hemolyzed sample or whole blood sample is usually in the Oxy-Hb state, unless the blood donor was recently exposed to carbon monoxide or the person suffers from methemoglobinemia. Exposure to carbon monoxide (mainly due to smoke inhalation) causes an elevation of Carboxy-Hb, and methemoglobinemia causes an elevation of Met-Hb. Oxidation of the iron in the heme moiety of Hb molecules, is a normal process that occurs *in vivo*. Enzymes are continually at work reversing the process and thus preventing the accumulation of Met-Hb (for example, NADH methemoglobin reductase, and the met-Hb reductase system). Methemoglobinemia is a condition of people that lack enzymes required to reverse this oxidation process. Absence of the enzymes that reverse the oxidation process, also results in spontaneous oxidation of Hb to Met-Hb in hemolyzed serum or plasma over time, causing the sample to darken in the color.

Figure 3 shows how the absorbance spectra of a hemolyzed sample changes as it ages. The absorbance peak at about 632nm that accompanies the darkening of color indicates a conversion of Hb to Met-Hb. Accumulation of Met-Hb could also occur in serum or plasma of patients infused with Hb-based blood substitutes. A calibration algorithm for Met-Hb in hemolyzed serum or plasma samples or in a serum or plasma sample from a patient infused with Hb-based blood substitutes can therefore be developed, preferably using the negative absorbance slope of the peak with an absorbance maximum at about 630nm. Example 6 (Equation 22) gives an example of a primary calibration algorithm for Met-Hb, which uses 645nm as the principal calibration wavelength.

The measurement of Met-Hb as described herein, is used in the measurement of an indicator of hemolysis in serum, plasma, urine, cerebrospinal fluid, lymphatic fluid and synovial fluid, for measuring the oxidation of Hb into Met-Hb, and also for measuring the oxidation of Hb-based blood substitutes into their Met-Hb form. Also, the measurement of Met-Hb as described herein, is used in the measurement ofHb in whole blood of patients who are or who are not infused with Hb-based blood substitutes.

## OXIDATION OF HEMOGLOBIN

Oxidation of the iron in the heme moiety of Hb molecules is a normal process that occurs in vivo. Enzymes are continually at work reversing the oxidation process and thus preventing the accumulation of Met-Hb. Methemoglobinemia is a condition of people that lack enzymes, e.g., NADH methemoglobin reductase, required to reverse the oxidation process. The Met-Hb reductase system may be underdeveloped in infants, making methemoglobinemia more prevalent among infants. Another reason for the higher incidence of methemoglobinemia among infants and neonates is an underdeveloped gastrointestinal system in some infants. In an underdeveloped gastrointestinal system, bacteria level could rise due to a decrease secretion of gastric acid. Nitrates are usually converted into nitrites by bacteria of the gastrointestinal system, and the nitrites in turn react with the Hb to produce Met-Hb.

Lack of Met-Hb reductase enzymes in hemolyzed serum causes spontaneous oxidation of Hb to Met-Hb over time, causing the sample to darken in the color. With reference to Figure 3, the absorbance peak at about 632nm that accompanies the darkening of color and that indicates a conversion of Hb to Met-Hb can be observed. Accumulation of Met-Hb could also occur in patients who are not lacking the Met-Hb reductase enzymes. In these patients, the accumulation of Met-Hb could be induced by the intake of certain therapeutic drugs and other chemicals, for examples, which should not be considered limiting in any way: dapsone, chloroquine, phenazopyridine, phenacetin, nitrates, nitrites, phenols, and aniline. Patients with high levels of Met-Hb, whatever the cause, should be monitored for the increase of Met-Hb, or the decrease of Met-Hb after treatment, or both the increase and decrease.

In a normal person, the composition of Hb (% of Tot-Hb) in the arterial blood is about 95 % Oxy-Hb, about 2% Deoxy-Hb, about 2% Carboxy-Hb and about 1% Met-Hb, as measured by CO-Oximetry. In a heavy smoker, the %Carboxy-Hb can be about 10%. It should be understood that the Hb composition depends on the CO-oximeters used to measure the % of the Hb species. Newer CO-oximeters tend to give different numbers, which are supposedly more reliable, since the measurements in the newer CO-oximeters are performed at more wavelengths. More wavelengths could help compensate for interfering substances like, for example, bilirubin, turbidity, Sulfhemoglobin, and fetal hemoglobin. It should also be understood that although CO-oximeters are considered by some as reference instruments for measuring the % Hb species, the methods using CO-oximeters are not true reference methods for measuring the % of the Hb species in a blood sample.

The Total-Hb and Met-Hb could be measured in a pinprick blood sample and the %Met-Hb calculated. The calibration algorithm for measurement of %Met-Hb could also be developed empirically by taking the ratio of absorbances of a sample at two different wavelengths, for example about 630nm and about 560nm. With reference to Figure 2, it can be noted that the absorbance at about 630nm is greater for Met-Hb than for the same amount of each of the other species shown; the reverse is true at about 560nm. These wavelengths are just examples that can be used, and should not be considered limiting in any way.

Furthermore, the ratio of absorbances at 560nm and 940 nm, could be one of more than one ratio term in a calibration algorithm for %Met-Hb. It should be understood, that the use of ratio of absorbances as a single term, or the use of the sum of more than one similar term in a calibration algorithn is preferred. However, any statistical technique used to develop a calibration algorithm is considered to be within the scope of the present invention. A method for correcting the measurement ofTot-Hb (used as an indicator of hemolysis in serum and plasma), for the presence of Met-Hb is disclosed in US 6,689,612 (Samsoondar; which are incorporated herein by reference). The methods described for calibration of Met-Hb, correcting Total-Hb for the presence of Met-Hb, and flagging Total-Hb for the presence of Met-Hb, could also be used for whole blood samples.

## DEGRADATION AND REVERSAL OF DEGRADATION OF HEMOGLOBIN-BASED BLOOD SUBSTITUTES

Blood transfusion is a life-saving process that is performed after severe blood loss after trauma or during surgery. Some advantages of using a blood substitute instead of whole blood (by "whole blood" it is meant the combination the cellular and non-cellular components of blood) or red blood cells are as follows:

a) blood substitutes are expected to be universally compatible with all blood types, therefore cross matching would not be necessary;

b) maximum storage time of blood is 42 days, whereas the blood substitutes could have a much longer shelf life; and

c) the purification process of the blood substitute may include heat treatment, which can minimize the threat of hazardous viruses.

Most blood substitutes under development are made from human Hb, bovine Hb, or recombinant DNA technology (recombinant Hb). Hemoglobin comprises four protein subunits, which are two pairs of identical polypeptide chains. Each subunit has a molecular weight of about 16,000, with a cleft that contains a heme (iron-porphyrin) group, the site of oxygen uptake. The subunits are not covalently linked, and require the red cell membrane to keep the subunits together. A hemoglobin molecule is too large to penetrate the kidney, but the subunits are small enough to become lodged in the kidney and cause kidney failure.

In Hb-based blood substitutes, the subunits of the Hb could be chemically cross-linked with each other or to large polymers, or the Hb molecules could be linked to other Hb molecules to form poly-Hb, for stability. The Hb subunits may be inter- or intra-molecularly cross-linked. Regardless of the protein or polymer surrounding the heme groups, the absorbance spectrum of Hb-based blood substitutes is almost identical to normal Hb, but subtle differences at certain wavelengths may be present. The Hb-based blood substitutes are not protected from uncontrollable spontaneous oxidation into Met-Hb since they are no longer housed within the red cell membrane, where the Hb is usually in contact with Met-Hb reductase enzymes. A detailed review of blood substitutes is provided in volumes I and II of "Blood Substitutes: Principles, Methods, Products and Clinical Trials" (1998, by T.M.S. Chang, published by Karger Landes Systems). It should be understood that any form of Hb-based blood substitutes is considered to be within the scope of the present invention.

Due to the absence of the Met-Hb reductase enzymes, accumulation of Met-Hb could occur in the plasma of patients infused with Hb-based blood substitutes. Measurement or calculation of the ratio of Met-Hb to Total-Hb is useful for monitoring the degradation of Hb-based blood substitutes to its Met-Hb form, or for monitoring the reversal of degradation (e.g. degradation due to oxidation) process after for example, administration of one or more therapeutic agents, or monitoring a retardation in the spontaneous oxidation process by encapsulating the Hb-based blood substitutes with enzymes like NADH methemoglobin reductase or other reducing agents. In this example, the two blood analytes are the Hb-based blood substitute, and the Met-Hb form of the Hb-based blood substitute. In a patient infused with one or more types of Hb-based blood substitutes, it should be understood that the Total-Hb could include both the one or more Hb-based blood substitutes and endogenous Hb, and the Met-Hb could include both the Met-Hb forms of the one or more Hb-based blood substitutes and endogenous Met-Hb.

A method for monitoring degradation (e. g., oxidation) of Hb-based blood substitutes requires development of calibration algorithms for Met-Hb and the Hb-based blood substitute. The calibration algorithms can be developed by optionally using any statistical technique to process EMR absorbed by a sample at one or more wavelengths. The concentration of the one or more Hb-based blood substitutes and the Met-Hb can then be determined by applying the respective calibration algorithm to the absorbance of the sample at one or more wavelengths. Using a calibration algorithm for Met-Hb and another calibration algorithm for the Hb-based blood substitute, will allow the Met-Hb to be reported as a proportion, fraction, or percent of the total Hb-based blood substitute. Alternatively, a calibration algorithm could be developed for the proportion, fraction, or percent of the total Hb-based blood substitute, which is in the form of Met-Hb.

US Patent Application No. 10/136,329 (Publication Number 2003-0138960 Al; Samsoondar; which are incorporated herein by reference), describes a method of monitoring the degradation of Hb-based blood substitutes by monitoring the production of the Met-Hb derivative of the Hb-based blood substitutes. The application teaches that the sample can be whole blood, serum, plasma, or a body part from the patient infused with the blood substitute. The same method can also be used to monitor degradation of stock Hb-based blod substitutes. By a "Stock Hb-based blood substitute," it is meant a manufactured Hb-based blood substitute that is ready for use, for example, which should not be considered limiting in any way, for infusion by a patient. A method for correcting the measurement of Tot Hb (used as an indicator of hemolysis in serum and plasma), for the presence of Met-Hb is disclosed in US 6,689,612 (Samsoondar; which are incorporated herein by reference).

The above description is not intended to limit the claimed invention in any manner. The present invention will be further illustrated in the following examples.

**Examples: Primary Calibration**

Primary calibration of an apparatus is a cumbersome, time intensive exercise because it requires the measurements of absorbance of a relatively large set of samples, referred to as primary calibration sets. The samples in the primary calibration set should be real or very close to real samples. Preferably, samples include all the absorbance variability expected in a sample, whereby the sample variability becomes built into the primary calibration algorithm. Vessels also contribute variability, and it is possible to develop one or more primary calibration algorithm using a combination of more than one vessel, whereby the vessel variability becomes built into the primary calibration algorithm. However, development of primary calibration algorithms that are specific to a particular type of vessel is preferred. The apparatus on which primary calibration is performed is referred to as the "First Apparatus". Another apparatus that uses a primary calibration algorithm or a modified form of the primary calibration algorithm, without the second apparatus itself undergoing the process of primary calibration, is referred to as a "Second Apparatus".

A primary calibration algorithm can be obtained as follows: Absorbance spectra are obtained for several samples that cover a concentration range of a given analyte for which the primary calibration algorithm is being developed. It is preferred that the samples include all the absorbance variability expected in a sample, whereby the sample variability becomes built into the primary calibration algorithm. A multiple linear regression is then performed to develop a linear combination having the order derivative of absorbance at specific wavelengths as the independent variable, and the concentration of the analyte as the dependent variable. Other statistical methods, for example simple linear regression that uses only one wavelength, partial least squares (PLS) and principal component analysis (PCA), may also be used. The equation thus obtained is a primary calibration algorithm.

Zero order derivative of absorbance (also referred to as raw absorbance) or any order derivative of absorbance may be used in the calibration process with second order derivative of absorbance being preferred, and first order derivative of absorbance being more preferred. One exception is for a simulator of turbidity (for example IL), where both zero order derivative of absorbance and the first derivative of absorbance are preferred. With respect to a lipid emulsion, for example IL, for samples in containers that attenuate light in a reproducible manner, zero order derivative of absorbance is preferred over first order derivative of absorbance, because the resulting primary calibration algorithm covers a wider analytical range i.e. a wider range wherein the relationship between the predicted values and actual concentrations of a lipid emulsion, for example IL, is linear. For samples in, for example, blood bag tubing, which may or may not contain black writing in the light path, as discussed in US Patent No. 6,268,910 B1, the first order derivative of absorbance is preferred.

Software tools used for developing primary calibration algorithms may comprise but are not limited to the following:

- Matlab™ used to create programs for smoothing absorbances and obtaining derivative of absorbances.

- MS Excel™ may be used to develop macros for calculating derivative of absorbances; StatView™ used to create algorithms by a process called "step-wise multiple linear regression." In the step-wise linear regression, the order derivative of absorbance measurements for all the wavelengths is presented to the Statview™ program; only the wavelengths at which the order derivative of absorbance contribute to the calibration fit at a predetermined level of significance are selected for the algorithms.

- Pirouette™ may be used to create calibration algorithms by PLS or PCA, using the measurements for all the wavelengths, or selected sections of the absorbance spectra.

It will be appreciated however that other software tools may also be used. It will also be appreciated that any statistical technique may be used for the preparation of a calibration algorithm, for example, which should not be considered limiting in any way, simple linear regression, multiple linear regression, and multivariate data analysis.

Examples of multivariate data analysis, which should not be considered limiting in any way, are Principal Component Analysis (PCA), Principal Component Regression (PCR), Partial Least Squares regression (PLS), Neural Networks and Genetic Algorithms Software tools used for developing primary calibration algorithms may comprise, but are not limited to the following:

- Matlab™ used to create programs for smoothing absorbances and obtaining order derivative of absorbances.

- MS Excel™ may be used to develop macros for calculating order derivative of absorbances;

- StatView™ used to create algorithms by a process called "step-wise multiple linear regression." In the step-wise linear regression, the order derivative of absorbance measurements for all the wavelengths is presented to the StatView™ program; only the wavelengths at which the order derivative of absorbance contribute to the calibration fit at a predetermined level of significance are selected for the algorithms.

- Pirouette™ may be used to create calibration algorithms by PLS or PCA, using the measurements for all the wavelengths, or selected sections of the absorbance spectra.

- Calibration algorithms may also include the techniques of Neural Network and Genetic Algorithms, although any statistical technique is considered to be within the scope of the present invention.

It will be appreciated however that other software tools may also be used. Many examples of the primary calibration procedure, in respect of blood analytes, are shown in the references incorporated within this application. It will be appreciated that a primary calibration algorithm may contain from a single wavelength term, in the simplest case, to multiple terms that use many wavelengths. Primary Calibration Algorithms can be obtained by a process of simple linear regression, multiple linear regression, multivariate analysis or a combination thereof. Some examples of multivariate analysis are PLS, PCA, Genetic Algorithm, and Neural Network.

It should be understood that any order derivative of absorbance can be used, and it should also be understood, that the robustness of a primary calibration algorithm depends on the inclusion of interfering substances in the primary calibration sets, one expect to encounter in real samples. The chemometrics methods referred to should not be considered limiting in any way, and any form of chemometrics and data processing are within the scope of the present invention.

It will also be appreciated that determination of analyte concentration in a sample in a second apparatus may be accomplished by using data pre-processing, including smoothing, calculation of first and higher order derivative of absorbance, interpolation of absorbances, multiplicative scatter correction, or data transformation. Similar data pre-processing may also be used prior to primary calibration algorithm development. Photometric correction may also be used on second apparatus depending on the required accuracy of the predicted value of an analyte concentration.

Any other methods of primary calibration algorithm development and any form of data transformation are within the scope of this invention. Example of data transformation, which should not be considered limiting in any way, include determining the log and anti-log of the analyte concentration, and Fourier transformation, which are well known to those skilled in the art (for example see Osborne, B.G., Fearn, T & Hindle, P.H., Practical NIR Spectroscopy with Applications in Food and Beverage Analysis, 1993, Published by Longman Scientific & Technical, which is incorporated herein by reference).

The primary calibration algorithms can vary in robustness, depending on the make up of the primary calibrators. Once a primary calibration algorithm has been obtained for a given analyte, the concentration of the analyte in a sample (i.e. a predicted value) can be determined by obtaining an absorption spectrum of the sample and applying the primary calibration algorithm for the analyte. Primary calibration algorithms for any number of analytes can be installed in an apparatus, and they can be applied to the same absorbance data, in order to obtain concentrations of the analytes. Furthermore, more than one primary calibration algorithm can be installed for one analyte. The use of multiple primary calibration algorithms may be used to extend the analytical range of the spectroscopic apparatus at higher or lower analyte concentrations.

It should be understood that the analytes disclosed herein are by way of example only, and they should not limit the use of the apparatus of the present invention in any way.

## DEVELOPMENT OF PRIMARY CALIBRATION ALGORITHMS

The examples given below mostly describe analytes in plasma. However, it should be understood that similar methods of calibration algorithm development for analytes in other types of samples, for example, which should not be considered limiting in any way, calibrators, whole blood, serum, plasma, urine, synovial fluid, lymphatic fluid, sputum, feces, dairy products, beverages, a body part, for example but not limited to, a finger, arm, ear lobe, or a pharmaceutical tablet which should not be considered limiting in any way, are within the scope of the present invention.

To prepare a primary calibration algorithm for hemoglobin, sixty serum specimens with no visible interferents were stored refrigerated or frozen until used. More or fewer specimens may be used so long as a sufficient number is used to provide robust algorithm(s). Hemoglobin (Hb), INTRALIPID (IL), bilirubin (BR) and biliverdin (BV) were added to the normal sera to give final concentrations of 0-6.1 g/L, 0-5.1 g/l, 0-42.7 mg/dL, and 0-4.4 mg/dL respectively. Stock Hb was prepared by replacing the plasma (free from all interferents) from a blood sample, with twice its volume of water, and lysing the cells through three freeze-thaw cycles. For each cycle the blood was left in the freezer for 45 - 60 minutes, and then removed and placed on a rocker at room temperature for 30 - 45 minutes.

Hb content of the lysate was measured by a spectroscopic method for measuring oxy-Hb described by Tietz ((Tietz Textbook of Clinical Chemistry, 3rd Ed, 1999, pp 1674-1676), after removing the RBC debris and unlysed RBC's by centrifuging at 10,000 x g for 10 minutes. Any method that provides a reliable determination of Hb content may be used. A typical hemolysate contains approximately 100 g/L Hb. CO-oximetry suggests that more than 95% of the Hb is in the oxy-Hb state. Stock BV was prepared by dissolving biliverdin dihydrochloride (obtained from Sigma) initially in 50% methanol-50% water, and diluting further with phosphate buffered saline (PBS). Stock IL also known as TRAVAMULSION™ (preferably obtained from Clintec-Nestle & Baxter) has a concentration of 10%. Stock BR was prepared by dissolving Ditauro-Bilirubin (from Porphyrin Products, Logan, Utah, USA) in interferent-free serum, to a concentration of 500 mg/dL. The spectral absorbance data were recorded for the 60 samples using different polypropylene dispensing tips. Out of the 60 samples, odd numbers were used for the calibration set, and even numbers were used as the validation set. This primary calibration set does not contain Met-Hb or MB, therefore these substances may contribute to inaccuracies in the Hb measurements. Met-Hb and MB may be included in the absorbance variability of the primary calibration set, in order to obtain more robust primary calibration algorithms.

A summary of exemplary primary calibration algorithms, which are not to be considered limiting in any manner, using the methods as described herein are presented in Table 1. It is to be understood that other primary calibration algorithms may be readily obtained using different substances, or sample containers, etc, and using the methods as described herein

Table 1:

| Wavelengths used in primary calibration algorithms shown in Examples 1 to 7, arranged according to analyte. | | | | | |
|---|---|---|---|---|---|
| Equation No. | Analyte | Wavelengths (nm) | | | |
| | | 1 | 2 | 3 | 4 |
| 1 | Hb | 584 | 599 | 617 | - |
| 2 | Hb | 600 | 618 | - | - |
| 3 | Hb | 591 | 653 | - | - |

Table 1: (continued)

| Wavelengths used in primary calibration algorithms shown in Examples 1 to 7, arranged according to analyte. | | | | | |
|---|---|---|---|---|---|
| Equation No. | Analyte | Wavelengths (nm) | | | |
| | | 1 | 2 | 3 | 4 |
| 4 | Hb | 600 | 663 | - | - |
| 5 | Hb | 558 | 570 | 730 | - |
| 6 | Hb | 591 | 610 | - | - |
| 7 | Hb-based Blood Substitute | 541 | 558 | 600 | 616 |
| 8 | BV | 649 | 731 | 907 | - |
| 9 | BV | 724 | 803 | - | - |
| 10 | BV | 718 | 781 | - | - |
| 11 | BR | 524 | 587 | 602 | - |
| 12 | BR | 534 | 586 | - | - |
| 13 | BR | 504 | 518 | 577 | - |
| 14 | BR | 495 | 512 | 578 | - |
| 15 | BR | 511 | 554 | - | - |
| 16 | IL | 700 | - | - | - |
| 17 | IL | 872 | - | - | - |
| 18 | IL | 988 | 1038 | - | - |
| 19 | IL | 874 | - | - | - |
| 20 | IL | 874 | - | - | - |
| 21 | IL | 900 | - | - | - |
| 22 | Met-Hb | 645 | 669 | - | - |
| 23 | MB | 702 | 759 | - | - |
| 24 | MB | 677 | 953 | - | - |

Also, the lowest and highest wavelengths shown in Table 1 are 504nm and 1038nm respectively, but it should be understood that calibration wavelengths within the range of about 300nm to about 2500nm, or any wavelength range therebetween, are within the scope of this invention.

**Example 1: Calibration algorithms for Hb**

Examples of primary calibration algorithms for Hb using the method described in the present application are given below. It will be appreciated that the algorithms can differ when the conditions in which they are obtained differ. Although the examples below show "g/L Hb" as the dependant variable, it should be understood that the dependant variable could be any indicator of hemolysis related to Hb, for example, Total-Hb, Oxy-Hb and "Total-Hb minus Met-Hb." The true indicator of hemolysis depends on both the reference method used to measure the indicator of hemolysis, and the substances included in the primary calibration set. As another aspect of this invention, methods for making corrections to the indicator of hemolysis are described, and whether correction is performed on the indicator of hemolysis, or the value of the indicator of hemolysis is only flagged to indicator potential error in the value, depends on the required accuracy of the indicator of hemolysis. It should be understood that measurement of Hb in whole blood is considered to be within the scope of the present invention.

Equation 1 (obtained using disposable polypropylene dispensing tips)

g/L Hb = -16.81 (1st D A584) + 79.47 (1st D A599) - 60.95 (1st D A617) + 0.24

where (1st D A) is the first derivative of the absorbance measurement at the wavelength specified in nanometers.

Equation 2 (obtained using 12 mm disposable polypropylene tubes)

$$g/L\ Hb = 113.27\ (1st\ D\ A600) - 182.94\ (1st\ D\ A618) - 0.13$$

where (1st D A) is the first derivative of the absorbance measurement at the wavelength specified in nanometers.

The following other examples ofprimary calibration algorithms for Hb are described in US 6,268,910 B1 and 5,846,492,WO 98/39634 and WO 97/47972.

Equation 3 (obtained using blood bag tubing)

$$g/L\ Hb = 45.68\ (1st\ D\ A591) - 47.48\ (1st\ D\ A653) - 0.42$$

where (1st D A) is the first derivative of the absorbance measurement at the wavelength specified in nanometers.

Equation 4 (obtained using disposable plastic dispensing tips)

$$g/L\ Hb = 15.89\ (1st\ D\ A600) - 15.88\ (1st\ D\ A663) - 0.21$$

where (1st D A) is the first derivative of the absorbance measurement at the wavelength specified in nanometers.

Equation 5 (obtained using disposable plastic dispensing tips)

$$g/L\ Hb = 30.72\ (1st\ D\ A558) - 17.40\ (1st\ D\ A570) + 171.14\ (1st\ D\ A730) - 072$$

where (1st D A) is the first derivative of the absorbance measurement at the wavelength specified in nanometers.

Equation 6 (obtained using translucent pipette tips)

$$(g/L)\ Hb = 30.14\ (1^{st}\ D\ A591) - 27.98\ (610)$$

where (1st D A) is the first derivative of the absorbance measurement at the wavelength specified in nanometers.

**Example 2: Calibration algorithms for Hb-based blood substitutes**

The following is an example of a primary calibration algorithm for Hb-based blood substitute as described in WO 98/39634.

Equation 7 (obtained using disposable polypropylene dispensing tips)

$$g/L\ Hb\text{-based blood substitute} = 23.97\ (1st\ D\ A541) - 76.01\ (1st\ D\ A558) + 130.84\ (1st$$

$$D\ A600) - 113.61\ (1st\ D\ A616) + 0.30$$

where (1st D A) is the first derivative of the absorbance measurement at the wavelength specified in nanometers.

**Example 3: Calibration algorithms for Biliverdin**

The following examples of primary calibrations algorithms for biliverdin are described in US 6,268,910 B1 and 5,846,492 and WO 97/47972.

Equation 8 (obtained using blood bag tubing)

$$mg/L\ BV = -45.40\ (1st\ D\ A649) + 323.15\ (1st\ D\ A731\ ) - 493.79\ (1st\ D\ A907) - 1.14$$

where (1st D A) is the first derivative of the absorbance measurement at the wavelength specified in nanometers.

Equation 9 (obtained using disposable plastic dispensing tips)

$$\text{mg/L BV} = 98.07 \,(\text{1st D A724nm}) - 122.73 \,(\text{1st D A803nm}) + 0.07$$

where (1st D A) is the first derivative of the absorbance measurement at the wavelength specified in nanometers.

Equation 10 (obtained using translucent pipette tips)

$$\text{mg/dL BV} = 160.29 \,(\text{1}^{\text{st}}\text{ D A718}) - 206.15 \,(\text{1}^{\text{st}}\text{ D A781}) + 1.42$$

where (1st D A) is the first derivative of the absorbance measurement at the wavelength specified in nanometers.

**Example 4: Calibration algorithms for Bilirubin**

The sample set used for Hb calibration is not typically used for BR calibration, because the absorbance due to either Hb >4g/L or IL >4g/L, approaches the limit of the apparatus in the region around 524nm, a primary wavelength used for BR calibration. Instead, a separate set of 60 samples were prepared and tested. As will be readily appreciated by those skilled in the art, the sample set used for primary calibration should be of a size sufficient to include most of the variability encountered with actual patient samples, such as serum or plasma. The samples were prepared as before by adding Hb, IL, BR and BV to the normal sera to give final concentrations of 0-2.6 g/L, 0-3.6 g/l, 0-37 mg/dL, and 0-4.4 mg/dL respectively. The spectral absorbance data were recorded for the 60 samples using different polypropylene dispensing tips. Out of the 60 samples, odd numbers were used for the calibration set, and even numbers were used as the validation set. The stock interferents were prepared as described above for Hb, and the BR concentrations were adjusted by the factor 1.23. The 1.23 factor that was derived previously from the slope of the regression line obtained from a validation set using real icteric serum and plasma samples. Met-Hb and MB is not expected to interfere with BR predictions, but they can only help to create more robust primary calibration algorithms, if they were included in the absorbance variability of the primary calibration set.

Equation 11 (obtained using disposable polypropylene dispensing tips)

$$\text{mg/dL BR} = 293.1 \,(\text{1st D A524}) + 327.8 \,(\text{1st D A587}) - 451.7 \,(\text{1st D A602}) - 7.5$$

where (1st D A) is the first derivative of the absorbance measurement at the wavelength specified in nanometers.

Equation 12 (obtained using 12 mm disposable polypropylene tubes)

$$\text{mg/dL BR} = 406.04 \,(\text{1st D A534}) + 183.94 \,(\text{1st D A586}) - 2.27$$

where (1st D A) is the first derivative of the absorbance measurement at the wavelength specified in nanometers.

The following examples of primary calibrations algorithms for bilirubin are described in US 6,268,910 B1, US 5,846,492 and WO 97/47972.

Equation 13 (obtained using blood bag tubing)

$$\text{mg/dL BR} = -43.03 \,(\text{1st D A504}) + 252.11 \,(\text{1st D A518}) + 240.03 \,(\text{1st D A577}) - 2.89$$

where (1st D A) is the first derivative of the absorbance measurement at the wavelength specified in nanometers.

Equation 14 (obtained using disposable plastic dispensing tips)

$$\text{mg/dL BR} = -24.88 \,(\text{1st D A495}) + 201.61 \,(\text{1st D A512}) + 44.98 \,(\text{1st D A578nm}) - 6.48$$

where (1st D A) is the first derivative of the absorbance measurement at the wavelength specified in nanometers.

Equation 15 (obtained using translucent pipette tips)

$$\text{mg/dL BR} = 142.09 \,(\text{1}^{\text{st}}\text{ D A511}) + 89.9 \,(\text{1}^{\text{st}}\text{ D A554}) - 4.47$$

where (1st D A) is the first derivative of the absorbance measurement at the wavelength specified in nanometers.

**Example 5: Calibration algorithm for Turbidity**

Turbidity in serum and plasma is caused mainly by the presence of fat particles, particularly chylomicrons. INTRALIPID™ (IL) is a lipid emulsion that simulates naturally-occurring chylomicrons, and therefore may preferably be used to simulate turbidity in serum and plasma.

Samples used for Hb and BR calibration are preferably not used for IL calibration because the Hb stock solution contributes significant light scattering (like lipid particles) due to unlysed RBC's and RBC fragments. Centrifugation of the hemolysate was unable to remove all the unlysed RBC's and RBC fragments.

Forty samples of PBS (phosphate buffered saline) were spiked with 10% Intralipid™ to produce concentrations of 0-20 g/L. The spectral absorbance data were recorded for the 40 samples using different polypropylene dispensing tips. Out of the 40 samples, the odd numbers were used for the calibration set, and the even numbers were used as the validation set. Suitable wavelengths used for IL calibration are from about 700nm to about 1100nm.

Turbidity is measured in terms of equivalent IL concentration.

Equation 16 (obtained using disposable polypropylene dispensing tips)

$$\ln (g/L\ IL) = 1.867(A700) - 0.447(A700)^2 + 0.041(A700)^3 - 1.33$$

where (A) is the raw absorbance measurement at the wavelength specified in nanometers.

Equation 17 (obtained using 12 mm disposable polypropylene tubes)

$$g/L\ IL = 2.72\ (A872) - 3.88\ (A872)^2 + 1.70\ (A872)^3 + 0.19$$

where (A) is the raw absorbance measurement at the wavelength specified in nanometers.

The following examples of primary calibrations algorithms for IL are described in US 6,268,910 B1, US 5,846,492 and WO 97/47972.

Equation 18 (obtained using blood bag tubing)

$$g/L\ IL = 432.42\ (1st\ D\ A988) + 40.40\ (1st\ D\ A1038) + 0.04$$

where (1st D A) is the first derivative of the absorbance measurement at the wavelength specified in nanometers.

Equation 19 (obtained using blood bag tubing)

$$g/L\ IL = 305.78\ (1st\ D\ A874) + 1.12$$

where (1st D A) is the first derivative of the absorbance measurement at the wavelength specified in nanometers.

Equation 20 (obtained using disposable plastic dispensing tips)

$$g/L\ IL = 252.16\ (1st\ D\ A874nm) + 0.24$$

where (1st D A) is the first derivative of the absorbance measurement at the wavelength specified.

Equation 21 (obtained using translucent pipette tips)

$$g/L\ IL = 296.01\ (A900) - 0.04$$

where (A) is the raw absorbance measurement at the wavelength specified in nanometers.

Example 6: Calibration algorithms for Met-Hemoglobin

Twenty nine samples comprising fresh hemolysate that contained about 95% Oxy-Hb, Met-Hb, MB, BV and IL were used to calibrate an apparatus that used TEFLON™ sample holders. BR was not added to the samples because BR does not absorb light at the wavelengths used to calibrate for either Met-Hb or MB. The Met-Hb was obtained in lyophilized form from Sigma, and was reconstituted in phosphate buffered saline. As mentioned above, the primary calibrations described herein is exemplary of the work involved in developing primary calibration algorithms.

Equation 22 (obtained using TEFLON™ sample holders)

$$\text{g/L Met-Hb} = 69.88 \text{ (1st D A645)} + 53.15 \text{ (1st D A669)} - 1.17$$

where (1st D A) is the first derivative of the absorbance measurement at the wavelength specified.

**Example 7: Calibration algorithm for Methylene Blue**

Equation 23 (obtained using TEFLON™ sample holders)

$$\text{mg/L MB} = 162.53 \text{(1st D A702)} - 112.58 \text{(1st D A759)} - 1.17$$

where (1st D A) is the first derivative of the absorbance measurement at the wavelength specified.

The following example of a primary calibration algorithm for MB is described in US 6,268,910 B1.

Equation 24 (obtained using blood bag tubing)

$$\text{mg/L MB} = 56.04 \text{ (1st D A677)} + 267.21 \text{ (1st D A953)} + 4.49$$

where (1st D A) is the first derivative of the absorbance measurement at the wavelength specified in nanometers.

The primary calibration algorithms referred to herein are non-limiting examples obtained by a process of step-wise multiple linear regression. A primary calibration algorithm may be developed using an order derivative of absorbance of calibration samples, at one or more than one wavelength of a standard set of wavelengths, and a statistical technique selected from the group consisting of simple linear regression, multiple linear regression, and multivariate analysis, wherein the multivariate analysis is selected from the group consisting of partial least squares, principal component analysis, neural network, and genetic algorithm. It should be understood that any order derivative of absorbance can be used, for example as shown for IL (Example 5, equations 18-20). The robustness of a primary calibration algorithm depends on the inclusion of substances in the primary calibration sets that absorb or scatter light around the principal calibration wavelength(s). Furthermore, similar calibration algorithms for Total-Hb and Met-Hb can be developed for Total-Hb and Met-Hb in whole blood, using similar methods as described above, for developing the calibration algorithms in plasma.

A primary calibration algorithm can also be obtained as follows: Absorbance spectra are obtained for several samples that cover a concentration range of a given analyte for which the primary calibration algorithm is being developed. It is preferred that the samples include all the absorbance variability expected in a sample, whereby the sample variability becomes built into the primary calibration algorithm. A multiple linear regression is then performed to develop a linear combination having the order derivative of absorbance at specific wavelengths as the independent variable, and the concentration of the analyte as the dependent variable. Other statistical methods, for example simple linear regression that uses only one wavelength, partial least squares (PLS), principal component analysis (PCA), neural network, and genetic algorithm may also be used. The equation thus obtained is a primary calibration algorithm.

All citations are hereby incorporated by reference.

The present invention has been described with regard to one or more embodiments. However, it will be apparent to persons skilled in the art that a number of variations and modifications can be made without departing from the scope of the invention as defined in the claims.

**Claims**

1. A spectroscopic apparatus, comprising:

a) a source of electromagnetic radiation (EMR);

b) a first aperture located between the source of EMR and a sample slot to produce a light path therebetween;

c) the sample slot in the apparatus for receiving a sample vessel to be placed within the light path;

d) a second aperture located in the light path, between the sample slot and one or more than one photodetector, the one or more than one photodetector in operative association with the spectroscopic apparatus; and

e) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

2. The apparatus of claim 1, wherein the sample vessel is selected from the group consisting of, a cuvette, a sample tab, a pipette tip, tubing, a labeled test tube, an unlabeled test tube, blood bag tubing, a transparent sample container, a translucent sample container, and a flow-through cuvette.

3. The apparatus according to claim 2, wherein the sample tab contains the sample placed between a cover plate and a base plate, wherein at least a portion of the cover plate is transparent or translucent and at least a portion of the base plate is transparent or translucent, and wherein the cover plate is hingedly attached to the base plate.

4. The apparatus according to claim 1, wherein the source of EMR is selected from the group consisting of a tungsten lamp, one or more than one Light Emitting Diode (LED), and one or more than one laser.

5. The apparatus according to claim 1, wherein the one or more than one photodetector is selected from the group consisting of Photodiode or Charged Coupled Detector (CCD).

6. The apparatus according to claim 1, wherein the one or more than one photodetector is comprised of an array of detectors, housed inside a spectrometer within the spectroscopic apparatus, the spectrometer further comprising a diffraction grating.

7. The apparatus according to claim 1, wherein the one or more than one calibration algorithm was developed using an order derivative of absorbance of calibration samples, at one or more than one wavelength of a standard set of wavelengths, and a statistical technique selected from the group consisting of simple linear regression, multiple linear regression, and multivariate analysis.

8. The apparatus according to claim 7, wherein the multivariate analysis is selected from the group consisting of partial least squares, principal component analysis, neural network, and genetic algorithm.

9. The apparatus according to claim 1, wherein the sample is selected from the group consisting of whole blood, serum, plasma, urine, synovial fluid, lymphatic fluid, sputum, feces, cerebrospinal fluid and a non-biological sample.

10. The apparatus according to claim 1, wherein the one or more than one primary calibration algorithm is for an analyte selected from the group consisting of a Hb-based blood substitute, Total-Hb, Oxy-Hb, "Total-Hb minus Met-Hb," Met-Hb, bilirubin, biliverdin, methylene blue, and a combination thereof.

11. A spectroscopic apparatus comprising:

a) a source of electromagnetic radiation (EMR) for producing a light path;

b) an aperture located in the light path between the source of EMR and a sample slot;

c) a sample slot for receiving a sample vessel and placed within the light path, the sample slot comprising a first, and a second, side, the first side facing the source of EMR;

d) a reflective member positioned at or near the second side, the reflective member for reflecting the EMR that passes through the sample slot to produce a reflected light path;

e) one or more than one photodetector located within the reflected light path, the one or more than one photodetector in operative association with the spectroscopic apparatus;

f) one or more than one primary calibration algorithm in operative association with the spectroscopic apparatus, the one or more than one primary calibration algorithm developed using one or more than one other apparatus, or one or more than one upgraded primary calibration algorithm in operative association with the spectroscopic apparatus.

12. The apparatus of claim 11, wherein the sample vessel is selected from the group consisting of, a cuvette, a sample tab, a pipette tip, tubing, a labelled test tube, an unlabeled test tube, blood bag tubing, a transparent sample container, a translucent sample container, and a flow-through cuvette.

13. The apparatus according to claim 12, wherein the sample tab contains the sample placed between a cover plate and a base plate, wherein at least a portion of the cover plate is reflective, transparent or translucent and at least a portion of the base plate is reflective, transparent or translucent, and wherein the cover plate is hingedly attached to the base plate.

14. The apparatus according to claim 11, wherein the source of EMR is selected from the group consisting of a tungsten lamp, one or more than one Light Emitting Diode (LED), and one or more than one laser.

15. The apparatus according to claim 11, wherein the one or more than one photodetector is selected from the group consisting of Photodiode or Charged Coupled Detector (CCD).

16. The apparatus according to claim 11, wherein the one or more than one photodetector is comprised of an array of detectors, housed inside a spectrometer within the spectroscopic apparatus, the spectrometer further comprising a diffraction grating.

17. The apparatus according to claim 11, wherein the one or more than one calibration algorithm was developed using an order derivative of absorbance of calibration samples, at one or more than one wavelength of a standard set of wavelengths, and a statistical technique selected from the group consisting of simple linear regression, multiple linear regression, and multivariate analysis.

18. The apparatus according to claim 17, wherein the multivariate analysis is selected from the group consisting of partial least squares, principal component analysis, neural network, and genetic algorithm.

19. The apparatus according to claim 11, wherein the sample is selected from the group consisting of whole blood, serum, plasma, urine, synovial fluid, lymphatic fluid, sputum, feces, cerebrospinal fluid and a non-biological sample.

20. The apparatus according to claim 11, wherein the one or more than one primary calibration algorithm is for an analyte selected from the group consisting of a Hb-based blood substitute, Total-Hb, Oxy-Hb, "Total-Hb minus Met-Hb," Met-Hb, bilirubin, biliverdin, methylene blue, and a combination thereof.

21. A spectroscopic apparatus comprising:

    a) a source of electromagnetic radiation (EMR) for producing a light path;

    b) an aperture located within the light path between the source of EMR and a sample slot;

    c) the sample slot for receiving a sample vessel, and placed within the light path;

    d) one or more than one photodetectors located on a same side of the sample slot as the source of EMR, the one or more than one photodetector in operative association with the spectroscopic apparatus;

    e) one or more than one primary calibration algorithm in operative association with the spectroscopic apparatus, the one or more than one primary calibration algorithm developed using one or more than one other apparatus, or one or more than one upgraded primary calibration algorithm algorithm in operative association with the spectroscopic apparatus.

22. The apparatus according to claim 21, wherein the sample vessel is selected from the group consisting of, a cuvette, a sample tab, a pipette tip, tubing, a labelled test tube, an unlabeled test tube, blood bag tubing, a transparent sample container, a translucent sample container, and a flow-through cuvette.

23. The apparatus according to claim 22, wherein the sample tab contains the sample placed between a cover plate and a base plate, wherein at least a portion of the cover plate is reflective, transparent or translucent and at least a portion of the base plate is reflective, transparent or translucent, and wherein the cover plate is hingedly attached to the base plate.

24. The apparatus according to claim 21, wherein the source of EMR is selected from the group consisting of a tungsten lamp, one or more than one Light Emitting Diode (LED), and one or more than one laser.

25. The apparatus according to claim 21, wherein the one or more than one photodetector is selected from the group consisting of Photodiode or Charged Coupled Detector (CCD).

26. The apparatus according to claim 21, wherein the one or more than one photodetector is comprised of an array of detectors, housed inside a spectrometer within the spectroscopic apparatus, the spectrometer further comprising a diffraction grating.

27. The apparatus according to claim 21, wherein the one or more than one calibration algorithm was developed using an order derivative of absorbance of calibration samples, at one or more than one wavelength of a standard set of wavelengths, and a statistical technique selected from the group consisting of simple linear regression, multiple linear regression, and multivariate analysis.

28. The apparatus according to claim 27, wherein the multivariate analysis is selected from the group consisting of partial least squares, principal component analysis, neural network, and genetic algorithm.

29. The apparatus according to claim 21, wherein the sample is selected from the group consisting of whole blood, serum, plasma, urine, synovial fluid, lymphatic fluid, sputum, feces, cerebrospinal fluid and a non-biological sample.

30. The apparatus according to claim 21, wherein the one or more than one primary calibration algorithm is for an analyte selected from the group consisting of a Hb-based blood substitute, Total-Hb, Oxy-Hb, "Total-Hb minus Met-Hb," Met-Hb, bilirubin, biliverdin, methylene blue, and a combination thereof.

# Figure 1

## Figure 2

**Figure 3**

# Figure 4a

430

440

442

444

450

# Figure 4b

444

430

440

442b

450

**Figure 5a**

540

542

544

**Figure 5b**

544

546

542b

540

542

548

## Figure 6a

## Figure 6b

## Figure7a

702
704
706
714
708
710
712
720
716
718

## Figure 7b

720
704
708
712
710
718

## Figure 8